(19) Europäisches Patentamt
European Patent Office
Office européen des brevets

(11) EP 2 046 340 B1

(12) **EUROPEAN PATENT SPECIFICATION**

(45) Date of publication and mention of the grant of the patent:
**04.09.2013 Bulletin 2013/36**

(21) Application number: **07836181.3**

(22) Date of filing: **20.07.2007**

(51) Int Cl.:
***A61K 31/553*** *(2006.01)* ***A61P 43/00*** *(2006.01)*

(86) International application number:
**PCT/US2007/016513**

(87) International publication number:
**WO 2008/011174 (24.01.2008 Gazette 2008/04)**

(54) **JAK INHIBITORS FOR TREATMENT OF MYELOPROLIFERATIVE DISORDERS**

JAK-HEMMER ZUR BEHANDLUNG MYELOPROLIFERATIVER ERKRANKUNGEN

INHIBITEURS DE LA JAK POUR LE TRAITEMENT DES SYNDROMES MYÉLOPROLIFÉRATIFS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.07.2006 US 832627 P**
**19.07.2007 US 880063**

(43) Date of publication of application:
**15.04.2009 Bulletin 2009/16**

(73) Proprietor: **Cephalon, Inc.**
**Frazer, PA 19355 (US)**

(72) Inventors:
- **DOBRZANSKI, Pawel**
  **Downingtown, PA 19335-4537 (US)**
- **RUGGERI, Bruce A.**
  **West Chester, PA 19380-3900 (US)**

(74) Representative: **Hallybone, Huw George et al**
**Carpmaels & Ransford LLP**
**One Southampton Row**
**London WC1B 5HA (GB)**

(56) References cited:
**WO-A-2004/108132** **WO-A-2007/061764**
**WO-A-2007/070444**

- **AMIN H M ET AL: "Characterization of apoptosis induced by protein kinase C inhibitors and its modulation by the caspase pathway in acute promyelocytic leukaemia" BRITISH JOURNAL OF HAEMATOLOGY, OXFORD, GB, vol. 110, no. 3, 2000, pages 552-562, XP002427381 ISSN: 0007-1048**
- **LEVIS MARK ET AL: "Pharmacokinetic and pharmacodynamic studies of lestaurtinib (CEP-701) and PKC-412: Cytotoxicity is often dependent on non-FLT3-mediated effects." BLOOD, vol. 106, no. 11, Part 1, November 2005 (2005-11), pages 692A-693A, XP009093903 & 47TH ANNUAL MEETING OF THE AMERICAN-SOCIETY-OF-HEMATOLOGY; ATLANTA, GA, USA; DECEMBER 10 -13, 2005 ISSN: 0006-4971**
- **LAIRD ET AL: "Small molecule tyrosine kinase inhibitors: clinical development of anticancer agents" EXPERT OPINION ON INVESTIGATIONAL DRUGS, ASHLEY PUBLICATIONS LTD., LONDON, GB, vol. 12, no. 1, January 2003 (2003-01), pages 51-64, XP002328206 ISSN: 1354-3784**
- **QUENTMEIER H ET AL: "JAK2 V617F tyrosine kinase mutation in cell lines derived from myeloproliferative disorders" LEUKEMIA (BASINGSTOKE), vol. 20, no. 3, March 2006 (2006-03), pages 471-476, XP002462938 ISSN: 0887-6924**
- **DOBRZANSKI PAWEL ET AL: "CEP-701 is a JAK2 inhibitor which attenuates JAK2/STAT5 signaling pathway and the proliferation of primary cells from patients with myeloproliferative disorders." BLOOD, vol. 108, no. 11, Part 1, November 2006 (2006-11), page 1026A, XP009093896 & 48TH ANNUAL MEETING OF THE AMERICAN-SOCIETY-OF-HEMATOLOGY; ORLANDO, FL, USA; DECEMBER 09 -12, 2006 ISSN: 0006-4971**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Richard Van Etten ET AL: "Focus on myeloproliferative diseases and myelodysplastic syndromes", Cancer Cell, 1 December 2004 (2004-12-01), pages 547-552, XP055046393, Retrieved from the Internet: URL:http://ac.els-cdn.com/S153561080400341 1/1-s2.0-S1535610804003411-main.pdf?_tid=0 ba14a64-3d5b-11e2-8620-00000aab0f02&acdnat =1354547382_6c80f1c8ec89940d75ceff83590b4 d a5 [retrieved on 2012-12-03]
- William Vainchenker: "A Unique Activating Mutation in JAK2 (V617F) Is at the Origin of Polycythemia Vera and Allows a New Classification of Myeloproliferative Diseases", Hematology, 1 January 2005 (2005-01-01), XP055046394, Retrieved from the Internet: URL: http://asheducationbook.hematologylibr ary.org/ content/2005/1/195.full.pdf#page=1 &view=FitH [retrieved on 2012-12-03]

## Description

## FIELD OF THE INVENTION

The invention relates to treatment of myeloproliferative disorders (MPDs) and myelodysplastic syndromes. Specifically, the invention relates to a fused pyrrolocarbazole compound for use In treatment of MPDs and myelodysplastic syndromes with a compound that is a JAK2 inhibitor.

## BACKGROUND OF THE INVENTION

Myeloproliferative disorders (MPDs) are clonal malignancies characterized by overproduction of one or more hematopoietic lineages with a relatively normal differentiation resulting in a hypercellular bone marrow (BM). MPDs are thought to arise in a single, multipotent progenitor or stem cell, which dominates BM and blood. Cultured hematopoietic progenitors from patients with MPDs display altered growth properties and growth factor independence. The molecular basis for certain MPDs had been unclear until a series of recent reports identified a single amino acid substitution V617F in the pseudokinase domain of JAK2 as a prevalent molecular lesion in polycythemia vera (PV), essential thrombocythemia (ET) and chronic idiopathic myelofibrosis (CIMF). This mutation was found in 75%-97% of patients with PV, and about 40%-50% of patients with ET and CIMF (James et al., 2005, Trends in Molecular Medicine 11, 546-554). Importantly, no other mutation has been found in autoinhibitory or kinase domains of 85 other kinases (Levine et a/., 2005, Cancer Cell 7, 387-397). In addition, the V617F mutation has been also identified in patients with myelodysplastic syndromes (MDS). Based on the predicted JAK2 structure, the V617F substitution disrupts an autoinhibitory interaction between the JH2 and kinase (JH1) domains of the protein. Consequently, V617F mutants were constitutively active and conferred growth factor independence and constitutive STAT5 activation when expressed in BaF3/EPOR cells (Levine et al., Cancer Cell 2005, 7, 387-397; Kralovics et al., 2005, N. Engl. J. Med. 352, 1779-1790)). Erythroid progenitors carrying the V617F mutation grew in absence of exogenous erythropoietin (EPO) and formed endogenous erythroid colonies (EEC), a hallmark of MPDs and siRNA-mediated inactivation of JAK2 reduced EEC formation. Finally, mice transplanted with murine bone marrow cells expressing the V617F mutant, but not a wild type JAK2, developed pathological features closely resembling PV in humans including strong elevation of hemoglobin/hematocrit, leukocytosis, hyperplasia of megakaryocytes, extramedullary hematopoiesis resulting in spleenomegaly and myelofibrosis of bone marrow (James et al., 2005, Nature 434, 1144-1148; Wernig et al., 2006, Blood 2006 Feb 14, [Epub ahead of print]). Clinically, the presence of the mutation in patients with CIMF was associated with a more aggressive disease and significantly poorer survival (Campbell et al., 2006, Blood. 2098-2100).

There is a need in the art to countetact the phenotype associated with the mutant or activated JAK2 and to treat myeloproliferative diseases and myelodysplastic syndromes associated with activation of JAK2.

## SUMMARY OF THE INVENTION

Receptor-linked tyrosine kinases (trk) are transmembrane proteins that contain an extracellular ligand binding domain, a transmembrane sequence, and a cytoplasmic tyrosine kinase domain. Tyrosine kinases function in cellular signal transduction. Cell proliferation, differentiation, migration, metabolism and programmed death are examples of tyrosine kinase-mediated cellular responses. JAK2 is a non-receptor tyrosine kinase.

It has been discoverer that JAK2 inhibitors may be used to treat myeloproliferative disorders and other diseases in which constitutive expression of JAK2 contributes to a pathological state.

Thus, the invention provides a composition containing a fused pyrrolocarbazole derivative for use in a method of treating myeloproliferative disorders and related disorders with said composition.

The myeloproliferative disorders and related disorders associated with the activation JAK2 which may be treated by the invention include, but are not limited to myeloproliferative diseases such as, for example, polycythemia vera (PV), essential thrombocythemia (ET), myelofibrosis with myeloid metaplasia (MMM) also called chronic idiopathic myelofibrosis (CIMF), unclassified myeloproliferative disorders (uMFDs), hypereosinophilic syndrome (HES), and systemic mastocytosis (SM).

In the invention, a therapeutically effective amount of the JAK2 inhibitor is administered to the subject For the average 70 kg adult, a dose regimen may be, for example, about 20 to about 120 mg, twice a day. In some embodiments the dose for the average adult is about 40 to about 100 mg, twice a day. In other embodiments the dose for the average adult is about 60 to about 80 mg, twice a day.

In the invention the activity of certain proteins is reduced in the presence of the fused pyrrolocarbazole derivative as compared with the absence of the fused pyrrolocarbazole. These proteins include, but are not limited to JAK2, STAT5, STAT3, SHP2, GAB2, AKT, and ERK.

## BRIEF DESCRIPTION OF THE DRAWINGS

**Figure 1** shows inhibition of JAK2 Kinase Activity by CEP-701.

**Figure 2** shows results of allele-specific PCR (panel A) and restriction enzyme analysis (panel B) which were performed to confirm presence of the V617F mutation in HEL 92 cells. K562 cells, which do not harbor this mutation served as a wild type control. **Panel A**: For allele-specific PCR, mutant-specific primers generated a 203 bp product (arrow) diagnostic of the mutation; a 364 bp product served as an internal control for PCR. M, Molecular weight markers; Lanes 1 and 2, HEL 92 cells showing the 364bp mutant and wild-type alleles and the 203bp mutant-specific allele. Lane 3, control K562 cells showing only the 364bp wild-type allele. **Panel B:** For restriction analysis, a PCR product encompassing the V617F mutation was generated from HEL92 and K562 DNAs and digested with BsaXI restriction enzyme. Undigested (denoted by "-") and BsaXI digested (denoted by "+") PCR products are shown M, molecular weight markers. The predicted restriction pattern was generated for K562 DNA. Two independent DNA preparations were tested for each cell line.

**Figure 3** shows the effects of CEP-701 on JAK2/STAT signaling in HEL 92 cells. HEL 92 cells were incubated for 24h with CEP-701 at 0.1$\mu$M, 0.3$\mu$M, 1.0$\mu$M and 3.0$\mu$M, as indicated. Effects on JAK2/STAT signaling were evaluated by western blot using phospho-specific STAT3 and STAT5 antibodies, as indicated or by immunoprecipitation/ western blot protocol (IP/WB) for JAK2: total JAK2 antibody was used for IP and phosphorylation was evaluated by WB using phosphotyrosine antibody. Expression of Bc1x1 was determined by western blot.

**Figure 4** shows the effects of CEP-701 on growth of HEL 92 cells. HEL 92 cells were incubated with increasing concentrations of CEP-701, as indicated, for 24h and 48h. Effects on cell growth were evaluated by MTS assay. Experiments were performed in 10% FCS (Panel A and B) or without serum (Panel C and D). **Panel A** shows results from a 24-hour incubation in the presence of 10% fetal bovine serum (FBS). **Panel B** shows results from a 48-hour incubation in the presence of 10%FBS. **Panel C** shows results from a 24-hour incubation without FBS. **Panel D** shows results from a 48-hour incubation without FBS. For Panels A-D, increasing amounts of CEP-701 were added to separate cultures (shown in hatched bars); untreated cells are shown in the first bar.

Figure 5 shows effects of CEP-701 on growth of HEL 92 cells with compound replenished every 24 hours. HEL 92 cells were incubated in 2% FCS with increasing concentrations of CEP-701, as indicated, for 72h. CEP-701 was replenished every 24h. Effects on cell growth were evaluated by MTS assay. Untreated cells are shown in the first bar.

Figure 6 shows effects of CEP-701 on induction of apoptosis in HEL 92 cells. HEL 92 cells were incubated with increasing concentrations of CEP-701, as indicated, for 24h, 48h and 72h. Induction of apoptosis was analyzed by histone/DNA release assay. **Panel A** shows a 24 hour assay, **Panel B** shows a 48 hour assay and **Panel C** shows a 72 hour assay.

**Figure 7** shows inhibition of STAT5 activation by CEP-701 in HEL 92 cells. HEL 92 cells were incubated with increasing concentrations of CEP-701 for 1h, 1.5h and 2.5h, as indicated. Whole cell extracts were prepared and effects on STAT5 phosphorylation were evaluated by western blot using specific antibodies. Bands were quantified and extent of phosphorylation was normalized to the total amount of STAT5 in a sample. Results are shown as percentage of the remaining STAT5 phosphorylation as compared to vehicle treated samples.

The average of 5 experiments is shown at the bottom. Based on 5 independent experiments, the $IC_{50}$ for STATS inhibition in HEL 92 cells was determined to be about 10 nM.

**Figure 8** shows inhibition of STAT3 activation by CEP-701 in HEL 92 cells. HEL 92 cells were incubated with increasing concentrations of CEP-701 for 1h, 1.5h and 2.5h, as indicated. Whole cell extracts were prepared and effects on STAT3 phosphorylation were evaluated by western blot using specific antibodies. Bands were quantified and extent of phosphorylation was normalized to the total amount of STAT3 in a sample. Results are shown as percentage of the remaining STAT3 phosphorylation as compared to vehicle treated samples. The average of 5 experiments is shown at the bottom. The $IC_{50}$ for STAT3 inhibition in HEL 92 cells is shown to be about 10 nM.

**Figure 9** shows the effects of human $\alpha$1-AGP on CEP-701-mediated inhibition of STAT5 activity in HEL 92 cells. HEL 92 cells were incubated for 1h with 10 mg/ml of $\alpha$1-AGP and with increasing concentrations of CEP-701, as indicated. Whole cell extracts were prepared and effects on STAT5 phosphorylation were evaluated by western blot using specific antibodies. Bands were quantified and extent of phosphorylation was normalized to the total amount of STAT5 in a sample. Results are shown as percentage of the remaining STAT5 phosphorylation as compared to vehicle treated samples. Coincubation with 1.0 mg/ml AGP shifted the $IC_{50}$ for STAT5 inhibition to 3 $\mu$M.

**Figure 10** shows the effects of CEP-701 (30 mg/kg) administered subcutaneously, twice a day) on growth of HEL 92 tumor xenografts. **Panel A:** growth of untreated tumors is shown by squares, while treated tumors are shown by triangles. **Panel B:** shows excised untreated and treated tumors.

**Figure 11** shows the effects of CEP-701 on JAK2/STAT signaling in HEL 92 tumor xenografts. A single dose of CEP-701 (30 mg/kg) was administered subcutaneously to animals carrying HEL 92 tumor xenografts and effects on STAT5 and STAT3 activation were evaluated at 0, 1, 2, 4, 8, and 12 hours after CEP-701 administration by Western blot. The intratumoral concentrations of CEP-701 were also determined.

**Figure 12** shows genotyping of clinical samples for the presence of the V617F mutation by allele-specific FCR. Patient #648 and #650 diagnosed with chronic idiopathic myelofibrosis (CIMF) were tested for the V617F mutation by allele-specific PCR. The wild-type and mutant alleles amplify as a 364bp fragment, while the 203bp, mutant-specific PCR product indicates presence of the V617F mutation. Also shown are results for HEL 92 (which contains the mutant allele) and the wild-type K562 cells.

**Figure 13** shows genotyping of clinical samples with restriction enzyme BsaX1. PCR product encompassing the V617F mutation was digested by BsaX1, as indicated. SLC is a JAK2-unrelated PCR product containing BsaX1 sites, which serves as a control of the completeness of the digestion.

**Figure 14** shows effects of CEP-701 on growth of CD34+ primary cultures derived from patient #648 (XTT assay). **Panel A:** 24 hours. **Panel B:** 48 hours.

**Figure 15** shows the effects of CEP-701 on growth and survival of CD34+ primary cultures derived from patient #648. CD34+ hematopoietic progenitors were purified from the patient #648 and cultured with increasing concentrations of CEP-701, as indicated. **Panel A;** cells were treated with CEP-701 for 24h and effects on cell growth (live cells) and apoptosis (Annexin V) were evaluated by FACS analysis. **Panel B:** cells treated for 24h with CEP-701 were diluted into a fresh medium and cultured for 72h. Cell count was determined using trypan blue. T=0 represents number of cells at the beginning of the experiment.

**Figure 16** shows the effects of CEP-701 on JAK2/STAT signaling in CD34+ primary cultures derived from Patient #648. CD34+ hematopoietic progenitors were purified from the patient #648 and cultured with increasing concentrations of CEP-701, as indicated, for 1h. Whole cell extracts were prepared and expression and phosphorylation of STAT5, STAT3 was analyzed by Western blot using specific antibodies. To evaluate JAK2 activity, JAK2 was immunoprecipitated, followed by a Western blot using antiphosphotyrosine specific antibody (4G10). The blot was stripped and reprobed with JAK2 antibody. Cyclophilin and β actin served as internal controls.

**Figure 17** shows the effects of CEP-701 on SHP2 and Gab2 activation and AKT and MAPK signaling in CD34+ primary cultures derived from Patient #648. CD34+ hematopoietic progenitors were purified from the patient #648 and cultured with increasing concentrations of CEP-701, as indicated, for 1h. Whole cell extracts were prepared and expression and phosphorylation of various signaling molecules was analyzed by western blot using specific antibodies. **Panel A:** Effects of CEP-701 on activation of SHP2 and GAB2 were evaluated by phosphospecific antibodies (pSHP2 and pGAB2). Total levels of expression were analyzed (SHP2 and GAB2). **Panel B:** Effects of CEP-701 on MAPK and AKT signaling were evaluated using phosphospecific antibodies against AKT (pAKT) and ERK (pERK). Total levels of expression were analyzed using specific antibodies (AKT and ERK). β actin served as internal control.

**Figure 18** shows the effects of 24h incubation with CEP-701 on STAT5 signaling in CD34+ primary cultures derived from Patient #648. CD34+ hematopoietic progenitors were purified from the patient #648 and cultured with increasing concentrations of CEP-701, as indicated, for 24h. Whole cell extracts were prepared and expression and phosphorylation of STAT5 and expression of Bc1x1 was analyzed by western blot using specific antibodies. Expression of β actin and cyclophilin served as internal control.

## DETAILED DESCRIPTION OF ILLUSTRATIVE EMBODIMENTS

The present invention relates to biologically active fused pyrrolocarbazole derivatives for use in methods for the treatment of various disorders or syndromes associated with JAK2 activation. In certain embodiments, the disorders and/or syndromes, include for example, myeloproliferative disorders (MPDs) and myelodysplastic syndromes. Among the fused pyrrolocarbazole derivatives useful for treatment of the disorders and/or syndromes are indolocarbazole and indenocarbazole derivatives. More specifically, the invention relates to an indolocarbazole for use in treatment of MPDs and myelodysplastic syndromes and other related disorders associated with JAK2 activation.

The fused pyrrolocarbazole derivatives useful in such disorders are known in the art and may be prepared in any number of ways by the skilled artisan, including, for example, U.S. Patent No. 4,923,986 to Murakata, et al.; 5,705,511 to Hudkins, et al.; 5,808,060 to Hudkins, et al.; U.S. Patent No. 6,127,401 to Singh, et al.; 6,841,567 U.S. Patent No. to Hudkins, et al.; 6,630,500 to Gingrich, et al.; 5,756,494 to Lewis, et al.; 5,468,872 to Glicksman, et al.; 5,516,771 to Dionne, et al.; 6,306,849 to Hudkins, et al.; and 6,093,713 to Hudkins, et al.; the disclosures of which are incorporated by reference herein in their entireties. In certain cases, the starting materials in the preparation of the indolocarbazoles, for example, K-252a and KT-5556, are optically active. The use of K252a or KT5556 as starting material in the preparation of compounds useful in methods of treatment herein described, may lead to partial or full transfer of the optical activity to the fused pyrrolocarbazole derivatives desired.

As employed above and throughout the disclosure, the following terms, unless otherwise indicated, shall be understood to have the following meanings.

As used herein, "about" refers to a range of values ± 10% of a specified value. For example, "about 20" includes ± 10% of 20, or from 18 to 22, inclusive.

As used herein, "alkyl" refers to an optionally substituted, saturated straight, or branched, hydrocarbon having from about 1 to about 8 carbon atoms (and all combinations and subcombinations of ranges and specific numbers of carbon atoms therein), preferably with from about 1 to about 4 carbon atoms (referred to herein as "lower alkyl"). Alkyl groups include, but are not limited to, methyl, ethyl, n-propyl, isopropyl, n-butyl, isobutyl, t-butyl, n-pentyl, isopentyl, neopentyl, n-hexyl, isohexyl, 3-methylpentyl, 2,2-dimethylbutyl, and 2,3-dimethylbutyl.

As used herein, "alkenyl" refers to an optionally substituted alkyl group having from about 2 to about 10 carbon atoms, more preferably having from about 2 to about 8 carbon atoms, and one or more double bonds (and all combinations and subcombinations of ranges and specific numbers of carbon atoms therein), wherein alkyl is as previously defined.

As used herein, "alkynyl" refers to an optionally substituted alkyl group having from about 2 to about 10 carbon atoms, more preferably having from about 2 to about 8 carbon atoms, and one or more triple bonds (arid all combinations and subcombinations of ranges and specific numbers of carbon atoms therein), wherein alkyl is as previously defined.

As used herein, "aryl" refers to an optionally substituted, mono-, di-, or tri-cyclic aromatic ring system having from about 6 to about 14 carbon atoms (and all combinations and subcombinations of ranges and specific numbers of carbon atoms therein), with from about 6 to about 10 carbons being preferred. Non-limiting examples include, for example, phenyl, naphthyl, anthracenyl, and phenanthrenyl.

As used herein, "arylalkyl" refers to an optionally substituted moiety composed of an alkyl radical bearing an aryl substituent, wherein the aralkyl moiety has from about 7 to about 22 carbon atoms (and all combinations and subcombinations of ranges and specific numbers of carbon atoms therein), with from about 7 to about 10 carbon atoms being preferred. Non-limiting examples include, for example, benzyl, diphenylmethyl, triphenylmethyl, phenylethyl, and diphenylethyl.

As used herein, "heteroaryl" refers to an optionally substituted aromatic ring system having 5 to 14 carbon atom ring members wherein one or more of the carbon atom ring members is independently replaced by one or more heteroatoms. In certain embodiments, the heteroatom is selected from S, O, or N. Heteroaryl groups having a total of from about 5 to about 14, more preferably from about 5 to about 6, carbon atom ring members and heteroatom ring members (and all combinations and subcombinations of ranges and specific numbers of carbon and heteroatom ring members) are preferred. Exemplary heteroaryl groups include, but are not limited to, pyrryl, furyl, pyridyl, pyridine-N-oxide, 1,2,4-thiadiazolyl, pyrimidyl, thienyl, isothiazolyl, imidazolyl, tetrazolyl, pyrazinyl, pyrimidyl, quinolyl, isoquinolyl, thiophenyl, benzothienyl, isobenzofuryl, pyrazolyl, indolyl, purinyl, carbazolyl, benzimidazolyl, and isoxazolyl. Heteroaryl groups may be attached via a carbon or a heteroatom to the rest of the molecule.

As used herein, "heteroarylalkyl" refers to an optionally substituted ring system composed of a heteroaryl substituted alkyl radical where heteroaryl and alkyl are as previously defined. Non-limiting examples include, for example, 2-(1H-pyrrol-3-yl)ethyl, 3-pyridylmethyl, 5-(2*H*-tetrazolyl)methyl, and 3-(pyrimidin-2-yl)-2-methylcyclopentanyl.

As used herein, the terms "Pro" "Ser" "Gly" "Lys" refer to the amino acids proline, serine, glycine, and lysine, respectively.

As used herein, the term "amino acid" denotes a molecule containing both an amino group and a carboxyl group. Embodiments of amino acids include α-amino acids; i.e., carboxylic acids of general formula $HOOC-CH(NH_2)$-(side chain). Side chains of amino acids include naturally occurring and non-naturally occurring moieties. Non-naturally occurring (i.e., unnatural) amino acid side chains are moieties that are used in place of naturally occurring amino acid side chains in, for example, amino acid analogs. *See,* for example, Lehninger, Biochemistry, Second Edition, Worth Publishers, Inc, 1975, pages 73-75, incorporated by reference herein. Preferred α-amino acids include glycine, alanine, proline, glutamic acid, and lysine, having the D configuration, the L configuration, or as a racemate.

As used herein, the term "Glc" refers to glucose.

As used herein, the term "substantially enriched", when referring to a stereoisomer or stereoisomeric center, denotes that at least about 60%, preferably about 70%, more preferably about 80%, still more preferably about 90% of one stereoisomer or one stereoisomeric center predominating in the mixture, with at least about 95% of one stereoisomer or one stereoisomeric center being even more preferred. In some preferred embodiments, the compound is "substantially enantiomerically pure", that is, at least about 97.5%, more preferably about 99%, even more preferably about 99.5% of one stereoisomeric forms predominates.

As used herein, the term "effective amount" refers to an amount of a compound as described herein that may be therapeutically effective to inhibit or treat the symptoms of particular disease, disorder, or condition. Such diseases, disorders, and conditions include, but are not limited to, those pathological conditions associated with the activity of JAK2, wherein the treatment comprises, for example, inhibiting the activity thereof by contacting cells, tissues or receptors with compounds of the present invention. Thus, for example, the term "effective amount," when used in connection with compounds of the invention for the treatment of myeloproliferative disorders, refers to the treatment of the symptoms,

diseases, disorders, and conditions typically associated myeloproliferative disorders.

As used herein, "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. These physiologically acceptable salts are prepared by methods known in the art, *e.g.*, by dissolving the free amine bases with an excess of the acid in aqueous alcohol, or neutralizing a free carboxylic acid with an alkali metal base such as a hydroxide, or with an amine. Compounds described herein throughout, can be used or prepared in alternate forms. For example, many amino-containing compounds can be used or prepared as an acid addition salt. Often such salts improve isolation and handling properties of the compound. For example, depending on the reagents, reaction conditions and the like, compounds as described herein can be used or prepared, for example, as their hydrochloride or tosylate salts. Isomorphic crystalline forms, all chiral and racemic forms, N-oxide, hydrates, solvates, and acid salt hydrates, are also contemplated to be within the scope of the present invention.

As used herein, "hydrate" refers to a compound of the present invention which is associated with water in the molecular form, i.e., in which the H-OH bond is not split, and may be represented, for example, by the formula $R\cdot H_2O$, where R is a compound of the invention. A given compound may form more than one hydrate including, for example, monohydrates ($R\cdot H_2O$) or polyhydrates ($R\cdot nH_2O$ wherein n is an integer > 1) including, for example, dihydrates ($R\cdot 2H_2O$), trihydrates ($R\cdot 3H_2O$), and the like, or hemihydrates, such as, for example, $R\cdot n_{/2}H_2O$, $R\cdot n_{/3}H_2O$, $R\cdot n_{/4}H_2O$ and the like wherein n is an integer.

As used herein, "solvate" refers to a compound of the present invention which is associated with solvent in the molecular form, i.e., in which the solvent is coordinatively bound, and may be represented, for example, by the formula R·(solvent), where R is a compound of the invention. A given compound may form more than one solvate including, for example, monosolvates (R·(solvent)) or polysolvates (R·n(solvent)) wherein n is an integer > 1) including, for example, disolvates (R·2(solvent)), trisolvates (R·3(solvent)), and the like, or hemisolvates, such as, for example, $R\cdot n_{/2}$(solvent), $R\cdot n_{/3}$(solvent), $R\cdot n_{/4}$(solvent) and the like wherein n is an integer. Solvents herein include mixed solvents, for example, methanol/water, and as such, the solvates may incorporate one or more solvents within the solvate.

As used herein, "acid salt hydrate" refers to a complex that may be formed through association of a compound having one or more base moieties with at least one compound having one or more acid moieties or through association of a compound having one or more acid moieties with at least one compound having one or more base moieties, said complex being further associated with water molecules so as to form a hydrate, wherein said hydrate is as previously defined and R represents the complex herein described above.

Compounds described herein throughout, can be used or prepared in alternate forms. For example, many amino-containing compounds can be used or prepared as an acid addition salt. Often such salts improve isolation and handling properties of the compound. For example, depending on the reagents, reaction conditions and the like, compounds as described herein can be used or prepared, for example, as their hydrochloride or tosylate salts. Isomorphic crystalline forms, all chiral and racemic forms, N-oxide, hydrates, solvates, and acid salt hydrates, are also contemplated to be within the scope of the present invention.

As used herein, "patient" refers to animals, including mammals, preferably humans.

As used herein, "prodrug" refers to compounds specifically designed to maximize the amount of active species that reaches the desired site of reaction, which are of themselves typically inactive or minimally active for the activity desired, but through biotransformation are converted into biologically active metabolites.

As used herein, the term "stereoisomers" refers to compounds that have identical chemical constitution, but differ as regards the arrangement of the atoms or groups in space.

As used herein, "N-oxide" refers to compounds wherein the basic nitrogen atom of either a heteroaryl ring or tertiary amine is oxidized to give a quaternary nitrogen bearing a positive formal charge and an attached oxygen atom bearing a negative formal charge.

As used herein, the terms "treatment" and "treating" as used herein include preventative (e.g., prophylactic), curative and/or palliative treatment.

When any variable occurs more than one time in any constituent or in any formula, its definition in each occurrence is independent of its definition at every other occurrence.

Combinations of substituents and/or variables are permissible only if such combinations result in stable compounds.

It is believed the chemical formulas and names used herein correctly and accurately reflect the underlying chemical compounds. However, the nature and value of the present invention does not depend upon the theoretical correctness of these formulae, in whole or in part. Thus it is understood that the formulas used herein, as well as the chemical names attributed to the correspondingly indicated compounds, are not intended to limit the invention in any way, including restricting it to any specific tautomeric form or to any specific optical or geometric isomer, except where such stereochemistry is clearly defined.

Accordingly, in certain embodiments, the present invention is directed to a JAK2 inhibitor for use in methods of treating myeloproliferative disorders comprising administering to a patient in need thereof, a therapeutically effective amount of said JAK2 inhibitor.

In a preferred embodiment, the JAK2 inhibitor is a fused pyrrolocarbazole that is substantially enantiomerically pure. The JAK2 inhibitor is:

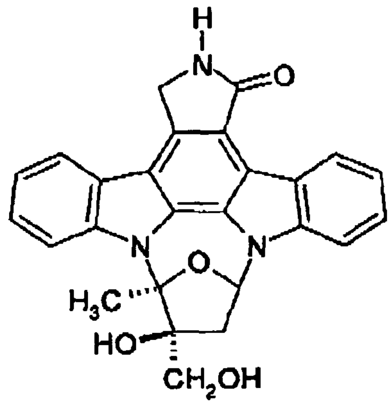

CEP-701

As used herein, the compound having this structure is referred to as "CEP-701."

The fused pyrrolocarbazole of the invention can be formulated into pharmaceutical compositions by admixture with pharmaceutically acceptable nontoxic excipients and carriers. Such compositions can be prepared for use in parenteral administration, particularly in the form of liquid solutions or suspensions; for oral administration, particularly in the form of liquid, tablets or capsules; or intranasally, particularly in the form of powders, natal drops, or aerosols.

The composition can be administered conveniently in unit dosage form and can be prepared by any of the methods known in the art. Such methods are described, for example, in Remington's Pharmaceutical Sciences (Mack Pub. Co., Easton, Pa., 1980).

Liquid dosage forms for oral administration include pharmaceutically acceptable emulsions, microemulsions, solutions, suspensions, syrups and elixirs. In addition to the active compound, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or (a) fillers or extenders such as starches, lactose, sucrose, glucose, mannitol, and silicic acid; (b) binders such as, for example, carboxymethylcellulose, alginates, gelatin, polyvinylpyrrolidinone, sucrose, and acacia; (c) humectants such as glycerol, (d) disintegrating agents such as agar-agar, calcium carbonate, potato or tapioca starch, alginic acid, certain silicates, and sodium carbonate; (e) solution retarding agents such as paraffin; (f) absorption accelerators such as quaternary ammonium compounds; (g) wetting agents such as, for example, cetyl alcohol and glycerol monostearate; (h) absorbents such as kaolin and bentonite clay; and (i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose

or milk sugar as well as high molecular weight polyethylene glycols and the like.

Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. In solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The optimal dosage of the compounds of the present invention may vary, depending on factors such as type and extent of progression of the myeloproliferative disorder, the overall health status of the patient, the age and weight of the patient, the potency of the compound, and the route of administration. Optimization of the compound dosage is within ordinary skill in the art and the values within the ranges provided here

include any value that falls within the range. The active ingredient in the formulations of the compound of the invention is effective when a concentration in the plasma is about 1 to about 20 μM including any value within this range, including, but not limited to 2, 3, 4, 5, 6, 7, 8, 9, 10, 11, 12, 13, 14, 15, 16, 17, 18, and 19 μM. In some preferred embodiments, the compound is administered to reach a concentration in the plasma of about 5 to about 15 μM. In other preferred embodiments, the compound is administered to reach a concentration in the plasma of about 7.5 to about 15 μM.

In certain embodiments of the present invention, the dosage of the compound is from about 200 μg/kg to about 1 g/kg body weight per day. More preferably, the dosage of the compound is from 250 μg/kg to about 3 mg/kg body weight per day. More preferably, the dosage of the compound is about 0.5 mg/kg to about 2.3 mg/kg body weight per day. More preferably, the dosage of the compound is about 0.8 mg/kg to about 1.3 mg/kg body weight per day.

In certain embodiments, the daily dose for the average adult (approx. 70 kg) is from about 20 mg to about 300 mg, or from about 40 mg to about 250 mg, or from about 40 to about 100 mg. More preferably, the daily dose is from about 80 mg to about 160 mg. The daily dose can typically be administered once a day, twice a day, three times a day or four times a day. In some embodiments the dosing regimen is from about 20 to about 120 mg, twice a day. In other embodiments, it is from about 20 to about 100 mg, twice a day. In further embodiments, the dose is about 60 to about 80 mg, twice a day.

In the invention, when a therapeutic amount of the JAK2 inhibitor is administered, the activity of a number of other proteins in the presence of the JAK2 inhibitor is less than the activity of the other proteins in the absence of the JAK2 inhibitor. These other proteins are, for example, a number of the following proteins; JAK2, STAT5, STAT3, SHP2, GAB2, AKT, and ERK.

The present invention is further illustrated by the following examples. The examples are provided for illustration purposes only, and they are not to be construed as limiting the scope or content of the invention in any way.

## EXAMPLES

### Patient's cells and cell lines

**HEL 92.1.7.** The HEL 92.1.7 human erythroleukemia cell line was purchased from ATCC (TIB-180) and cells were grown in RPMI 1640 medium with 2 mM L-glutamine adjusted to contain 1.5 g/L sodium bicarbonate, 4.5 g/L glucose, 10 mM HEPES, and 1.0 mM sodium pyruvate and fetal bovine serum, 10%, as recommended. CEP-701 or vehicle was added to a complete medium and incubated as indicated.

**CD34+.** Peripheral blood was collected from patients diagnosed with MPDs at the University of Pennsylvania Cancer Center after informed consent. Blood cells were separated by Ficoll gradient centrifugation (Pharmacia). The mononuclear cell layer was removed and washed. Mononuclear cells were incubated with anti-CD34 immunomagnetic beads and CD34+ cells were purified using the magnetic cell sorter AutoMacs (Miltenyi Biotech, all protocols with beads and device per manufacturer's recommendations). CD34+ cells were washed and counted. Cells were either used fresh or frozen as viable cells in 10% DMSO. CD34+ cells were cultured in IMDM media containing 20% serum substitute (BIT 9500, Stem Cell Technologies, Vancouver). Cells were incubated with stem cell factor, interleukin 6 and interleukin 3 for 3-5 days for initial expansion. After initial incubation, erythropoietin was added to culture media. Cells were further expanded for 2-5 days until adequate cells were available for experiments. All cytokines were obtained from R&D Systems.

Patients with Chronic Idiopathic Myelofibrosis (CIMF) were genotyped using the PCR and Restriction Digest methods described above. Patients #648 and #650 were found to carry the V617F mutation as shown by allele-specific PCR **(Fig. 12)** and BsaXI digestion **(Fig. 13).** In Fig. 14, SCL PCR product was used as a control. Upon digestion with BsaXI, the 496 bp SCL product is cut into 356 bp, 110 bp and 30 bp fragments.

**Example I**

**Inhibition of JAK2 Kinase Activity by CEP-701**

CEP-701 was tested for its ability to inhibit the kinase activity of baculovirus-expressed JAK2 in a microtiter plate assay utilizing time-resolved fluorescence (TRF) detection. The 96-well Costar high binding plates (Corning Costar #3922, Coming, NY) were first coated with 140 μL/well of 10 μg/mL Neutravidin (Pierce #31000, Rockford, IL) in Tris-buffered saline (TBS) at 37°C for 2 hours, followed by 100 μL/well of 1 μg/mL 15-mer peptide substrate (biotinyl-amino-bexanoyl-EQEDEPEGDYFEWLE-amide, Infinity Biotech Research and Resource, Aston, PA) at 37°C for another hour. The JAK2 assay mixture (total volume = 100 μL/well) consisting of 20 mM HEPES (pH 7.2), 0.2 μM ATP, 1 mM $MnCl_2$, 0.1 % bovine serum albumin (BSA), and varying concentrations of CEP-701 (diluted in DMSO; 2.5% DMSO final in assay) was then added to the assay plate. Enzyme (15 ng/ml JAK2 Lot # JAK2[318], JA2-2.1) was added and the reaction was allowed to proceed at room temperature for 20 minutes. Detection of the phosphorylated product was performed by adding 100 μL/well of Eu-N 1 labelled PY 100 antibody (PerkinElmer Life Sciences #AD0041, Boston, MA) diluted 1:5000 in TBS containing 0.05% Tween-20 and 0.25% BSA. Incubation at room temperature then proceeded for 1 hour, followed by addition of 100 μL enhancement solution (PerkinElmer Life Sciences #1244-105, Boston, MA). The plate was gently agitated and after thirty minutes, the fluorescence of the resulting solution was measured using the PerkinElmer EnVision 2100 (or 2102) multilabel plate reader. Inhibition curves were generated in replicates and plotted as percent inhibition versus log10 of the concentration of compound. Data were analysed by nonlinear regression by fitting to the sigmoidal dose-response (variable slope) equation in GraphPad Prism as follows:

$$y = bottom + (top - bottom)/(1 + 10 (\log IC_{50}-x)*Hillslope)$$

where y is the % inhibition at a given concentration of compound, x is the logarithm of the concentration of compound, bottom is the % inhibition at the lowest compound concentration tested, and top is the % inhibition at the highest compound concentration examined. The values for bottom and top were fixed at 0 and 100, respectively. $IC_{50}$ values from individual curves were averaged and reported as the mean $IC_{50}$ value. The concentration of CEP-701 versus JAK2 activity was plotted (Fig. 1). CEP-701 was shown to have an $IC_{50}$ of 0.9 ± 0.2 nM.

**Example 2**

**A. Allele-specific PCR for Val617Phe mutation**

Genotyping of cell lines and patient samples was performed as described in Baxter, E.J. et al. (2005) "Acquired mutation of the tyrosine kinase JAK2 in human myeloproliferative disorders" Lancet 365:1054-1061.

A dual approach, including allele-specific PCR and restriction enzyme analysis, was used to determine the status of the V617F mutation. Patients' DNA was prepared from granulocytes or mononuclear cells using Wizard Genomic DNA Puriftation Kit (Promega) following the manufacturer's instructions unless otherwise noted. PCR reactions were performed using PCR Core Kit from Promega according to the manufacturer's instructions unless otherwise noted.

Briefly, 80-200 ng of DNA was amplified in a PCR reaction (annealing temp. 58°C, 38-44 cycles) using the common reverse primer (5'-ctgaatagtcctacagtgttttcagtttca-3') (SEQ ID NO:1) and two forward primers. The first was a mutant specific primer (5'-agcatttggtntaaattatggagtatatt-3') (SEQ ID NO:2) that contains an intentional mismatch at the third nucleotide from the 3' end to improve specificity and which generates a 203 bp product indicating a presence of the V617F mutation. The second forward primer (5'-atctatagtcatgctgaaagtaggagaaag-3') (SEQ ID NO:3) amplifies a 364 bp fragment from mutated and wild type alleles and serves as an internal control. The results are shown in **Fig. 2** (Panel A). Both HEL 92 samples show amplification of the internal control as well as the 203 bp product from the mutant allele. K562 served as a wild-type control.

**B. Restriction Enzyme-Based Assay of *JAK2* Genotype**

The G→T mutation which results in the V617F substitution eliminates a restriction site for restriction enzyme BsaXI present in the wild type JAK2. Thus, resistance to BsaXI identifies the V617F mutation.

To assess the presence of the mutation in cell lines and Patient samples, 200 ng of genomic DNA encompassing the 617 position of JAK2 was amplified by PCR (57°C, 40-44 cycles) using the forward primer (5'-gggtttcctcagaacgttga-3') (SEQ ID NO:4) and the reverse primer (5'-tcattgctttcctttttcacaa-3') (SEQ ID NO:5). The resulting 460 bp fragment

was digested with BsaXI for 3-4 hours and analyzed on a 2% agarose gel. The wild type allele generates fragments of 241 bp, 189 bp and 31 bp, whereas the mutant allele remains intact. Amplified DNA fragments from HEL 92 cells were resistant to digestion with BsaXI, while amplified fragments from K562 cells were completely digested by BsaXI. To control the completeness of the digestion, a DNA fragment from a human SLC gene containing the BsaXI site was amplified and digested with BsaXI. The forward (5'-tcctggggtcttctgtcttg-3') (SEQ ID NO:6) and the reverse (5'-cctgagaggcaatgggagta-3') (SEQ ID NO:7) primers amplified a 496 bp SLC product, which was digested into 356 bp, 110 bp and 30 bp fragments. The results are shown in **Fig. 2** (Panel B).

**Example 3**

**Effects of CEP-701 on cell proliferation**

Purified and expanded CD34+ cells were counted and plated into 96 well plates for XTT assay. Cells were treated with either a JAK2 inhibitor or vehicle for 24-72 hours and relative cell viability was assayed using the XTT reagent (Molecular Probes) according to the manufacturer's recommendations.

HEL 92.1.7 cells ($2x10^4$/well) plated into 96 well plates were treated with CEP-701 or vehicle for 24-72 hours and cell viability was assayed by MTS reagent (Promega), as recommended.

Cell viability was assessed for HEL 92.1.7 cells treated with CEP-701 **(Fig. 4,** Panels A-D). In Panel A, cells were assessed at 24 hours post-incubation in the presence of fetal bovine serum (FBS) and upon treatment with CEP-701 in comparison to viability without treatment with drug). CEP-701 reduced the viability of cells down to about 50% (with 3.0 μM CEP-701). In Panel B, cells were assessed at 48 hours post-incubation in the presence of fetal bovine serum (FBS) and upon treatment with CEP-701 in comparison to viability without treatment with drug. CEP-701 reduced the viability of cells down to about 25% (with 3.0 μM CEP-701). In Panel C, cells were assessed at 24 hours post-incubation with CEP-701 without FBS. CEP-701 reduced the viability of cells down to about 50% (with 3.0 μM CEP-701) in a pattern similar to that in Panel A. In Panel D, cells were assessed at 48 hours post-incubation with CEP-701 without FBS. CEP-701 reduced the viability of cells down to about 25% (with 3.0 μM CEP-701) in a pattern similar to that shown in Panel B.

**Fig. 5** shows effects of CEP-701 on viability of HEL cells grown in the presence of fetal calf serum (FCS) with the drug replenished every 24h. In this experiment, HEL 92.1.7 cells were exposed to drug for 72 hours and assessed for viability in MTS assay. CEP-701 reduced viability of cells down to less than 5% (with 1.0 μM CEP-701).

The MTS assays indicate that CEP-701 inhibits growth of HEL 92 cells that carry the V617F mutation.

**Fig. 6** shows effects of CEP-701 on induction of apoptosis in HEL 92 cells as measured by histone/DNA release assay. The assay shows that CEP-701 induced apoptosis.

**Example 4**

**Analysis of effects of CEP-701 on signaling pathways**

Effects of CEP-701 on signaling pathways were evaluated by immunoprecipitation and/or Western blot using specific antibodies and whole cell extracts prepared from cell lines and cultured patient samples. Whole cell extracts were prepared in a lysis buffer: (20 mM Tris-HCl (pH 7.5), 150 mM NaCl, 1 mM $Na_2EDTA$, 1 mM EGTA, 1% Triton, 2.5 mM sodium pyrophosphate, I mM beta-glycerophosphate, I mM $Na_3VO_4$, 1 μg/ml leupeptin) supplemented with protease inhibitors (Complete Mini, Roche Diagnostic). Protein concentration was determined and equal amounts (10-30μg/lane) were separated on a SDS-PAGE gel. Proteins were transferred to a nitrocellulose filter and levels of expression and/or phosphorylation were assessed using specific antibodies, as recommended by manufacturers. Blots were developed using Supersignal West Pico System (Pierce) according to manufacturer's instructions. Activation of JAK2 was determined by an immunoprecipitation/Western blot protocol.

Briefly, JAK2 was immunoprecipitated from 250 μg of extract with JAK2 antibody using Immunoprecipitation Starter Pack (Amersham Biosciences) as recommended. Following Western blotting, tyrosine phosphorylation of JAK2 was evaluated by probing with phospho-tyrosine specific antibody 4G10 (Upstate).

**Fig. 3** Panel A shows a dose-dependent inhibition of phosphorylation of STATS by increasing concentrations of CEP-701 (from 0.1 to 3.0 μM CEP-701). Panel B shows inhibition of phosphorylation of JAK2 and STAT3 with increasing concentrations of CEP-701 (from 0.03 to 1.0 μM). Panel B also shows a dose-dependent inhibition of expression of $BclX_L$ (a downstream effector of JAK2/STAT5 signaling).

**Figure 7** shows inhibition of phosphorylation of STAT5 after 1.0, 1.5, and 2.5 hours of exposure to increasing concentrations of CEP-701. The average inhibition of 5 experiments is shown at the bottom. The $IC_{50}$ for STAT5 inhibition in HEL 92.1.7 cells is about 10 nM, which should translate to clinically meaningful suppression of JAK2/STAT signaling.

**Figure 8** shows inhibition of phosphorylation of STAT3 after 1.0, 1.5, and 2.5 hours of exposure to increasing

concentrations of CEP-701. The average inhibition of 5 experiments is shown at the bottom. The $IC_{50}$ for STAT3 inhibition in HEL 92.1.7 cells is about 10 nM, which should translate to clinically meaningful suppression of JAK2/STAT signaling.

**Figure 9** shows the effects of $\alpha$1-AGP on CEP-701-mediated inhibition of STAT5 activity in HEL 92 cells. As shown in the **Fig. 9,** 1.0 mg/ml of $\alpha$1-AGP shifted the $IC_{50}$ for STAT5 inhibition to 3 $\mu$M. These concentrations are readily achievable *in vivo* on an 80 mg dosing regimen, for example.

**Example 5**

**Effects of CEP-701 on the growth of HEL 92 Tumor Xenografts**

The effect of CEP-701 on the tumor growth was assessed (as compared to vehicle control) in mice bearing HEL 92 xenografts. Briefly, 30 mg/kg CEP-701 was administered subcutaneously twice a day and tumor volumes were assessed on selected days as shown in **Fig. 10.** Tumor growth was inhibited by CEP-701 and tumor volumes in CEP-701-treated mice were significantly reduced.

Effects of CEP-701 on JAK2/STAT signaling in HEL 92 xenografts were also evaluated. In this study animals bearing HEL.92 tumors received a single dose of CEP-701 (30mg/kg, s.c.) and kinetics of STAT5 and STAT3 inhibition was evaluated at different time points by Western blot. As demonstrated on **Fig. 11,** CEP-701 strongly suppressed STAT5 and STAT3 phosphorylation with maximum inhibition observed at 2-4h after the drug administration. The kinetics of inhibition correlated very well with CEP-701 concentration in tumors, further confirming ability of CEP-701 to suppress the JAK2/STAT signaling *in vivo.*

**Example 6**

**Patient Samples**

CEP-701 was shown to inhibit growth of CD34+ cells isolated from Patient #648 in an XTT assay **(Fig. 14).** Increasing concentrations of CEP-701 (0.03 $\mu$M, 0.1 $\mu$M and 0.3 $\mu$M) reduced growth of cells at 24 (Panel A) and 48 hours (Panel B).

**Fig. 15** shows a fluorescence activated cell sorter (FACS) analysis of CD34+ primary cultures derived from Patient #648 treated with CEP-701. In Panel A, the white bars indicate the number of live cells. The black bars in Panel A indicate induction of apoptosis as measured by Annexin V. In Panel B of **Fig. 15,** the black bars indicate the number of live CD34+ cells, which were treated for 24h with increasing concentrations of CEP-701 and than were diluted into a fresh medium and cultured for additional 72h. Cell count was determined using trypan blue.

**Figure 16** shows the effects of CEP-701 on JAK2/STAT signaling in CD34+ primary cultures derived from Patient #648. CD34+ hematopoietic progenitors were purified from the Patient #648 and cultured with increasing concentrations (30nM, 100nM, 300nM and 1000nM) of CEP-701, as indicated, for 1h. Expression and phosphorylation of STAT5, STAT3 was analyzed by Western blot using specific antibodies. To evaluate JAK2 activity, JAK2 was immunoprecipitated, followed by a Western blot using antiphosphotyrosine specific antibody (4G10). CEP-701 was shown to inhibit JAK2/STAT signaling in cultured CD34+ precursor cells carrying the V617F mutation and derived from a patient with MPD.

Effects of CEP-701 on activation of SHP2, GAB2, AKT and ERK were evaluated in cultured CD34+ cells derived from Patient #648. Western blot was used to examine phosphorylation and expression of SHP2, GAB2, **(Fig. 17,** Panel A) and AKT, and ERK **(Fig. 17,** Panel B). Increasing concentrations of CEP-701 were added to primary cultures of CD34+ cells derived from Patient #648. Concentrations used were 0 (*i.e.*, vehicle alone), 30, 100, 300 and 1000 nM of CEP-701. CEP-701 was shown to inhibit multiple signaling pathways involved in proliferation and survival of CD34+ precursor cells, suggesting a general shutdown at the receptor level in cells carrying the V617F mutation. In Panel B, expression of the housekeeping gene, $\beta$-actin, was used as a control.

Cells from Patient #648 were also assessed in experiment examining expression and phosphorylation of STAT5 and expression of $BCL\text{-}X_L$, a downstream target of JAK2/STAT signaling **(Fig. 18).** Increasing concentrations of CEP-701 were added to primary cultures of CD34+ cells derived from Patient #648. Concentrations used were 0 (*i.e.,* vehicle alone), 30, 100, 300 and 1000 nM of CEP-701. Expression of the housekeeping genes, $\beta$-actin and cyclophilin were used as a control. CEP-701 was shown to inhibit JAK2/STAT signaling and Bclx1 expression in these cells. Extended exposure to CEP-701 (*e.g.*, multiple dosing) may result in a downregulation of STATS expression.

As those skilled in the art will appreciate, numerous modifications and variations of the present invention are possible in light of the above teachings. It is therefore understood that within the scope of the appended claims, the invention may be practiced otherwise than as specifically described herein, and the scope of the invention is intended to encompass all such variations.

## Claims

1. A compound that is a JAK2 inhibitor for use in treating myeloproliferative disorders associated with activation of JAK2, wherein the compound has the structure:

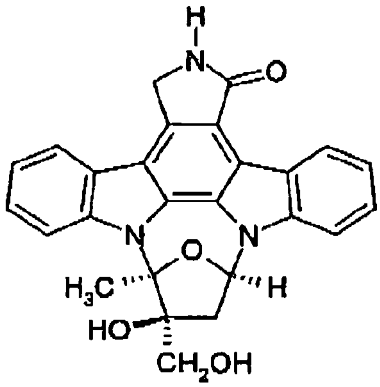

.

2. The compound as claimed in claim 1, wherein said myeloproliferative disorder is selected from the group consisting of polycythemia vera (PV), essential thrombocythemia (ET), myelofibrosis with myeloid metaplasia (MMM), idiopathic myelofibrosis (CIMF), unclassified mycloproliferative disorders (uMPDs), hypereosinophilic syndrome (HES), and systemic mastocytosis (SM).

3. The compound as claimed in claim 2, wherein the compound is administered in an amount of about 0.8 mg/kg body weight to about 1.3 mg/kg body weight per day.

4. The compound as claimed in claim 2, wherein the compound is administered in an amount of about 60 mg to about 80 mg twice a day.

5. The use of a compound that is a JAK2 inhibitor for the manufacture of a medicament for treating myeloproliferative disorders associated with activation of JAK2, wherein the compound has the structure:

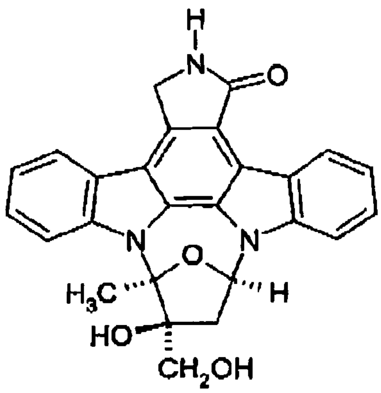

.

6. The use of claim 5, wherein said myeloproliferative disorder is selected from the group consisting of polycythemia vera (PV), essential thrombocythemia (ET), myelofibrosis with myeloid metaplasia (MMM), idiopathic myelofibrosis (CIMF), unclassified myeloproliferative disorders (uMPDs), hypereosinophilic syndrome (HES), and systemic mastocytosis (SM).

7. The use of claim 6, wherein the compound is adapted for administration in an amount of about 0.8 mg/kg body weight to about 1.3 mg/kg body weight per day.

8. The use of claim 6, wherein the compound is adapted for administration in an amount of about 60 mg to about 80 mg twice a day.

## Patentansprüche

1. Verbindung, bei der es sich um einen JAK2-Inhibitor handelt, zur Verwendung bei der Behandlung von mit der Aktivierung von JAK2 assoziierten myeloproliferativen Erkrankungen, wobei die Verbindung die folgende Struktur aufweist:

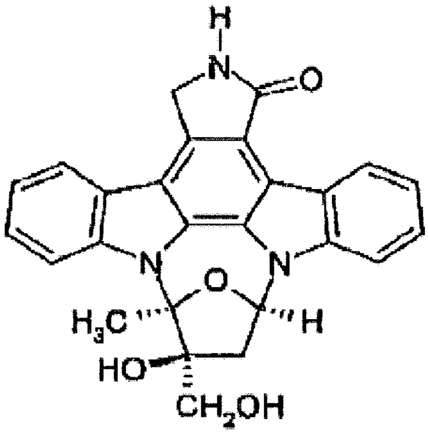

.

2. Verbindung nach Anspruch 1, wobei die myeloproliferative Erkrankung aus der aus Polycythemia vera (PV), essentieller Thrombozythämie (ET), Myelofibrose mit myeloider Metaplasie (MMM), idiopathischer Myelofibrose (CIMF), nichtklassifizierten myeloproliferativen Erkrankungen (uMPDs), hypereosinophilem Syndrom (HES) und systemischer Mastozytose (SM) bestehenden Gruppe ausgewählt ist.

3. Verbindung nach Anspruch 2, wobei die Verbindung in einer Menge von etwa 0,8 mg/kg Körpergewicht bis etwa 1,3 mg/kg Körpergewicht pro Tag verabreicht wird.

4. Verbindung nach Anspruch 2, wobei die Verbindung in einer Menge von etwa 60 mg bis etwa 80 mg zweimal täglich verabreicht wird.

5. Verwendung einer Verbindung, bei der es sich um einen JAK2-Inhibitor handelt, zur Herstellung eines Medikaments zur Behandlung von mit der Aktivierung von JAK2 assoziierten myeloproliferativen Erkrankungen, wobei die Verbindung die folgende Struktur aufweist:

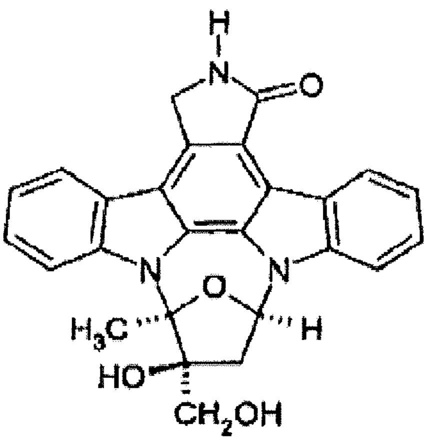

.

6. Verwendung nach Anspruch 5, wobei die myeloproliferative Erkrankung aus der aus Polycythemia vera (PV), essentieller Thrombozythämie (ET), Myelofibrose mit myeloider Metaplasie (MMM), idiopathischer Myelofibrose (CIMF), nichtklassifizierten myeloproliferativen Erkrankungen (uMPDs), hypereosinophilem Syndrom (HES) und systemischer Mastozytose (SM) bestehenden Gruppe ausgewählt ist.

7. Verwendung nach Anspruch 6, wobei die Verbindung für eine Verabreichung in einer Menge von etwa 0,8 mg/kg Körpergewicht bis etwa 1,3 mg/kg Körpergewicht pro Tag ausgelegt ist.

8. Verwendung nach Anspruch 6, wobei die Verbindung für eine Verabreichung in einer Menge von etwa 60 mg bis etwa 80 mg zweimal täglich ausgelegt ist.

**Revendications**

1. Composé qui est un inhibiteur de JAK2 pour utilisation dans le traitement de troubles myéloprolifératifs associés à l'activation de JAK2, le composé ayant la structure :

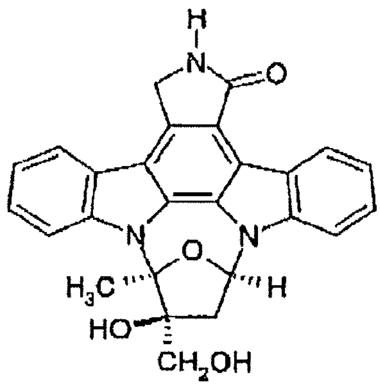

2. Composé selon la revendication 1, ledit trouble myéloprolifératif étant choisi dans le groupe constitué de la polycythémie vraie (PV), la thrombocythémie essentielle (ET), la myélofibrose avec métaplasie myéloïde (MMM), la myélofibrose idiopathique (CIMF), des troubles myéloprolifératifs non classifiés (uMPD), le syndrome hyperéosinophile (HES), et la mastocytose systémique (SM).

3. Composé selon la revendication 2, le composé étant administré en une quantité d'environ 0,8 mg/kg de poids corporel à environ 1,3 mg/kg de poids corporel par jour.

4. Composé selon la revendication 2, le composé étant administré en une quantité d'environ 60 mg à environ 80 mg deux fois par jour.

5. Utilisation d'un composé qui est un inhibiteur de JAK2 pour la fabrication d'un médicament pour traiter des troubles myéloprolifératifs associés à l'activation de JAK2, le composé ayant la structure :

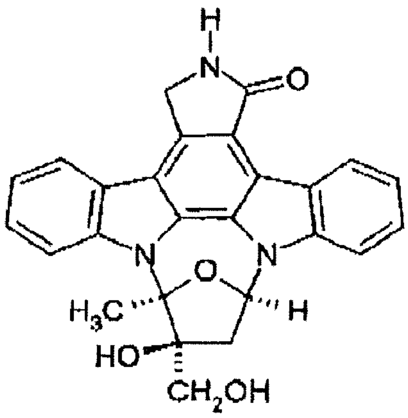

.

**6.** Utilisation de la revendication 5, ledit trouble myéloprolifératif étant choisi dans le groupe constitué de la polycythémie vraie (PV), la thrombocythémie essentielle (ET), la myélofibrose avec métaplasie myéloïde (MMM), la myélofibrose idiopathique (CIMF), des troubles myéloprolifératifs non classifiés (uMPD), le syndrome hyperéosinophile (HES), et la mastocytose systémique (SM).

**7.** Utilisation de la revendication 6, le composé étant adapté pour administration en une quantité d'environ 0,8 mg/kg de poids corporel à environ 1,3 mg/kg de poids corporel par jour.

**8.** Utilisation de la revendication 6, le composé étant adapté pour administration en une quantité d'environ 60 mg à environ 80 mg deux fois par jour.

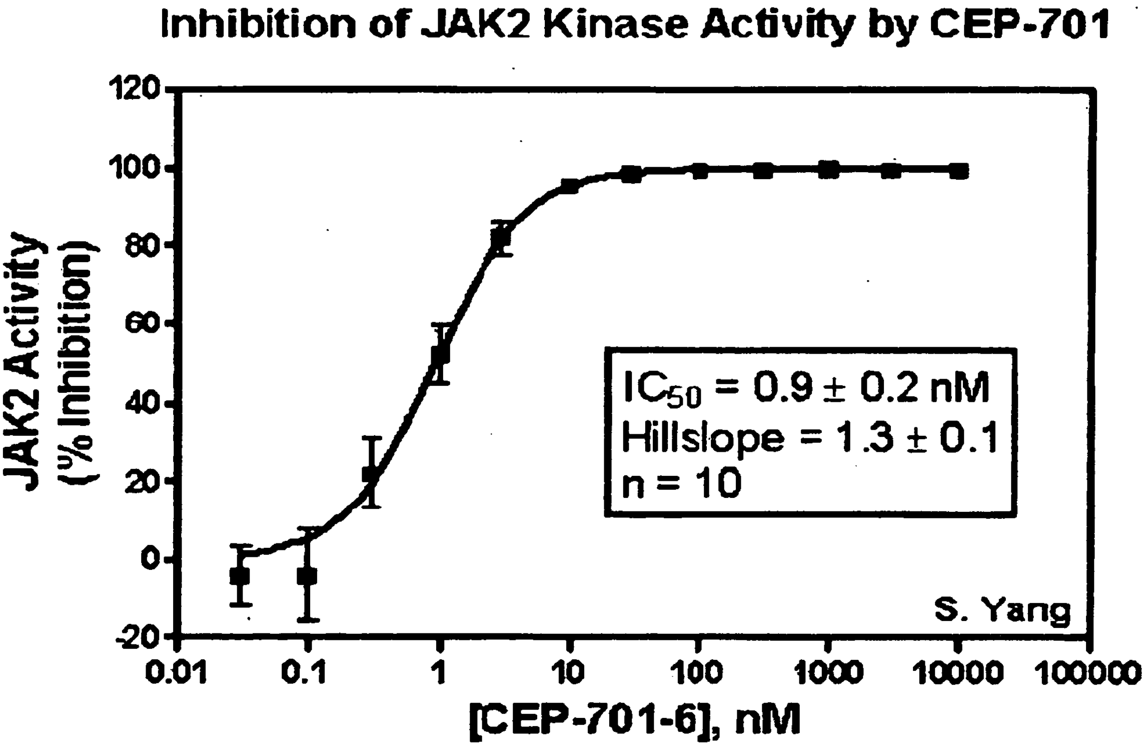
Inhibition of JAK2 Kinase Activity by CEP-701
JAK2 Activity
(% Inhibition)
120
100
80
60
40
20
0
-20
0.01
0.1
1
10
100
1000
10000
100000
[CEP-701-6], nM
IC50 = 0.9 ± 0.2 nM
Hillslope = 1.3 ± 0.1
n = 10
S. Yang

Figure 1

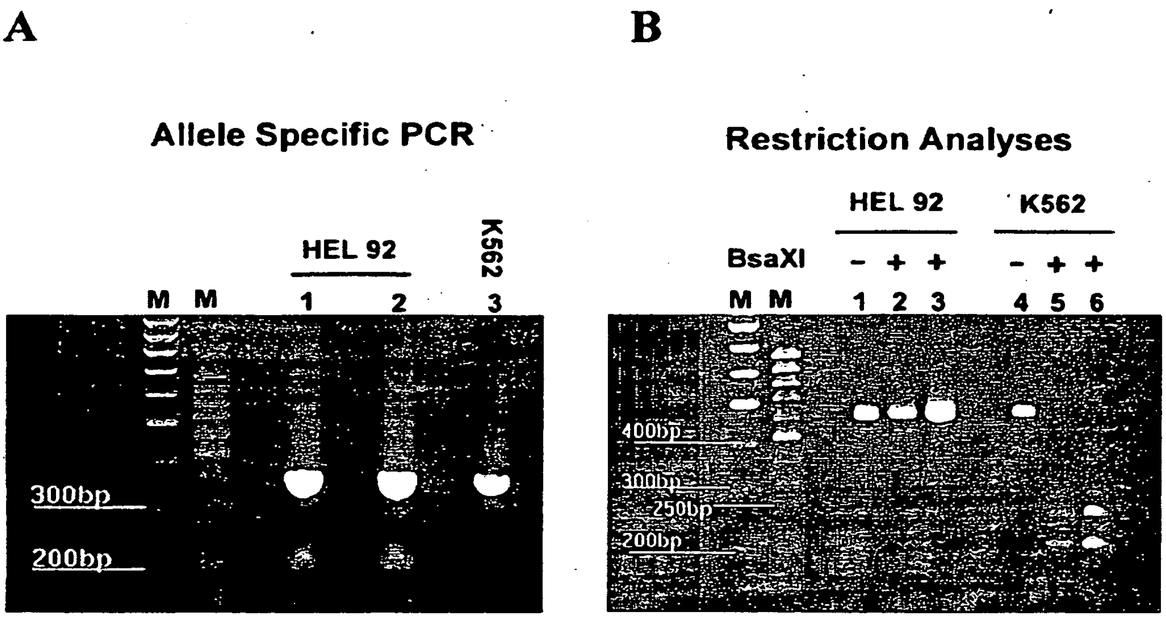


**364bp = mutant and wild type alleles**
**203bp =mutant allele**

**Figure 2**

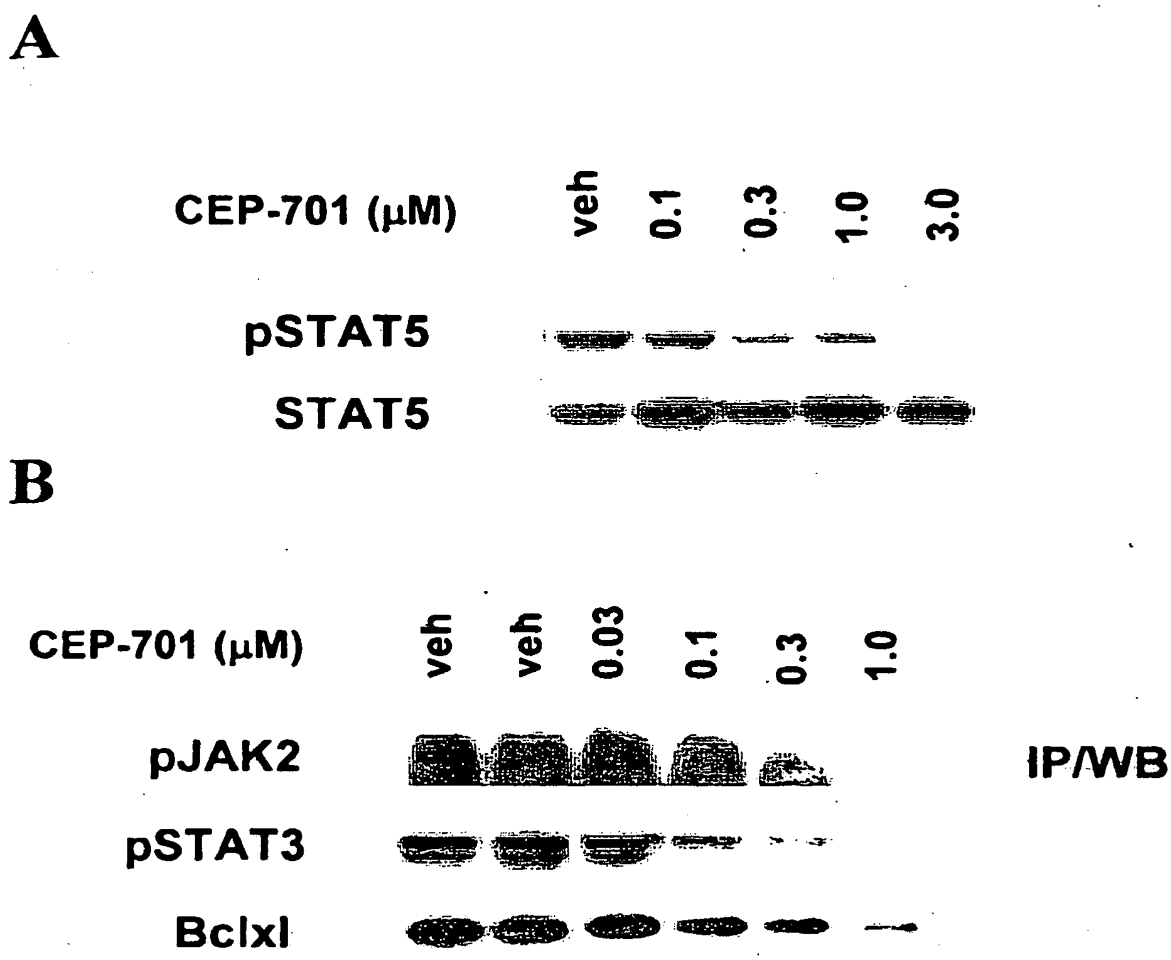
A
CEP-701 (μM)
veh
0.1
0.3
1.0
3.0
pSTAT5
STAT5
B
CEP-701 (μM)
veh
veh
0.03
0.1
0.3
1.0
pJAK2
pSTAT3
Bclxl
IP/WB

Figure 3

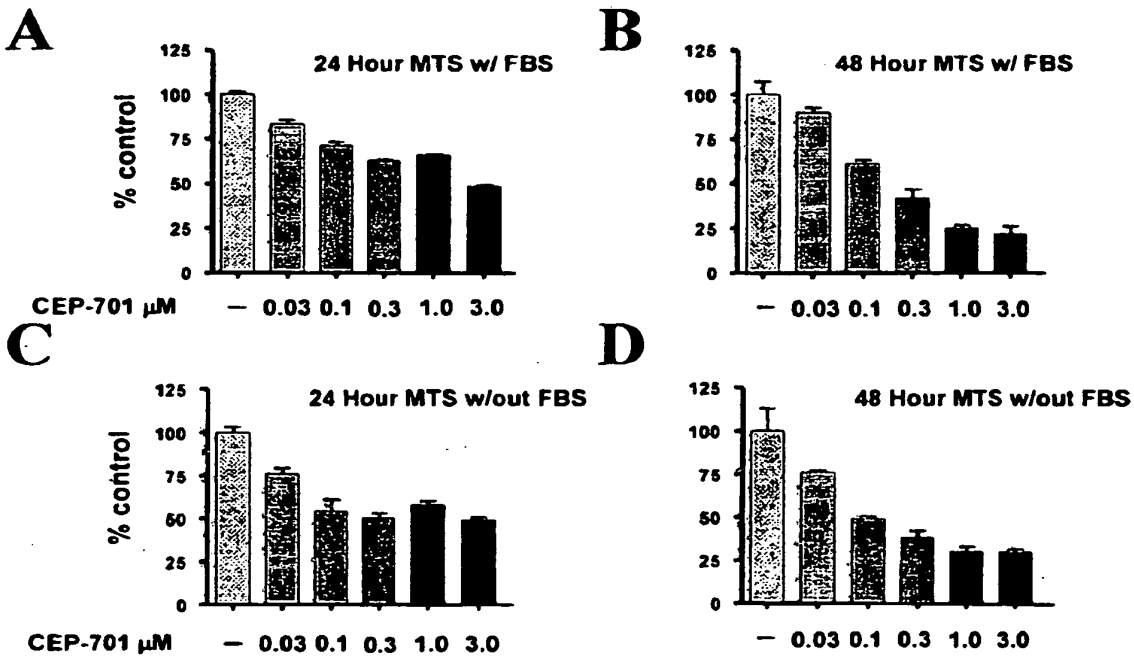
A
24 Hour MTS w/ FBS
% control
125
100
75
50
25
0
CEP-701 µM
— 0.03 0.1 0.3 1.0 3.0
B
48 Hour MTS w/ FBS
125
100
75
50
25
0
— 0.03 0.1 0.3 1.0 3.0
C
24 Hour MTS w/out FBS
% control
125
100
75
50
25
0
CEP-701 µM
— 0.03 0.1 0.3 1.0 3.0
D
48 Hour MTS w/out FBS
125
100
75
50
25
0
— 0.03 0.1 0.3 1.0 3.0

Figure 4

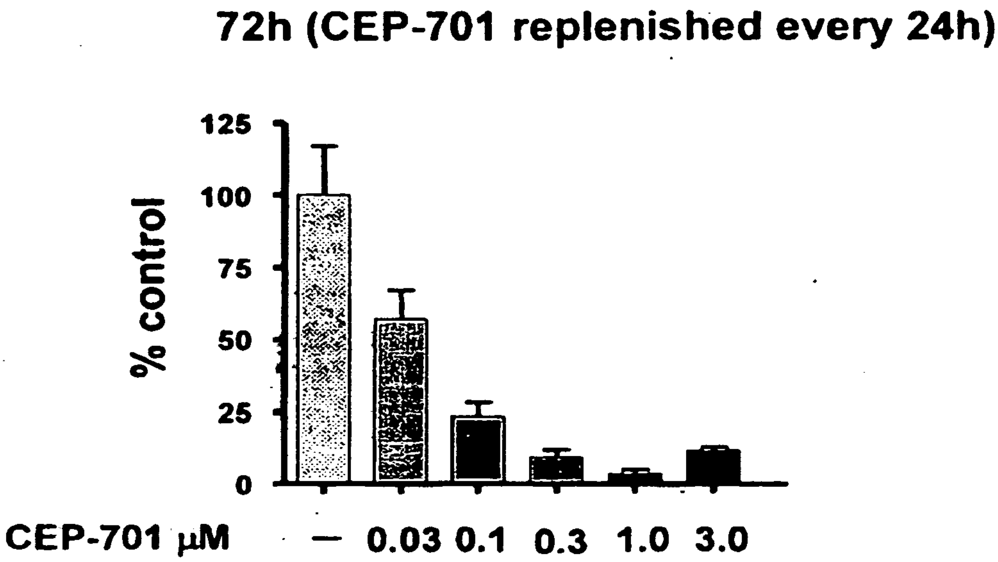
72h (CEP-701 replenished every 24h)
% control
125
100
75
50
25
0
CEP-701 μM
—
0.03
0.1
0.3
1.0
3.0

Figure 5

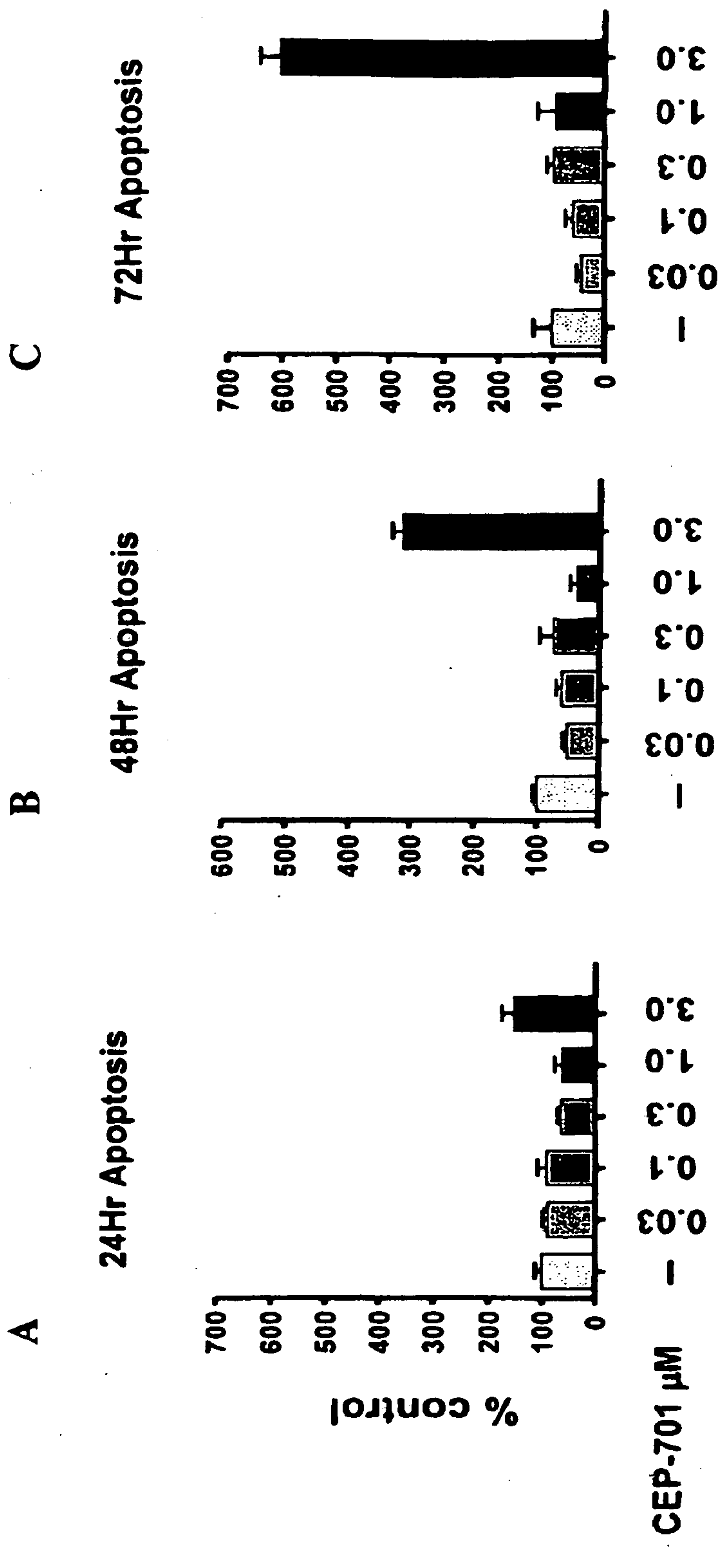
A
24Hr Apoptosis
B
48Hr Apoptosis
C
72Hr Apoptosis
% control
700
600
500
400
300
200
100
0
–
0.03
0.1
0.3
1.0
3.0
CEP-701 µM

Figure 6

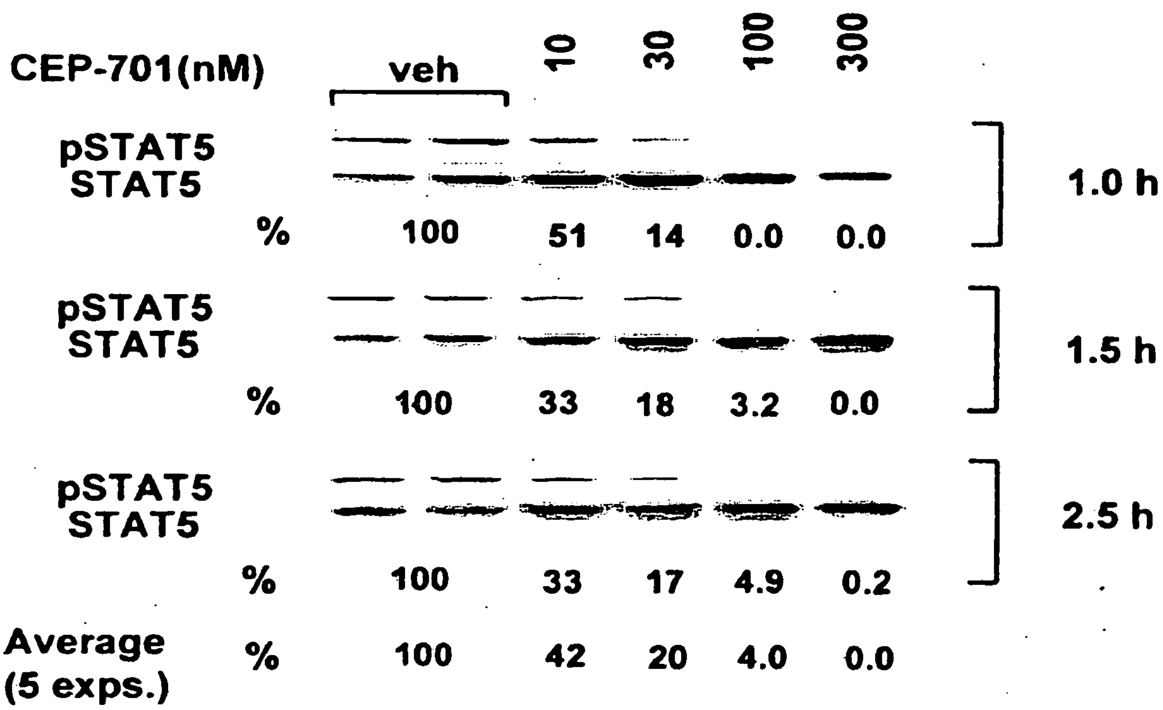
CEP-701(nM)
veh
10
30
100
300
pSTAT5
STAT5
%
100
51
14
0.0
0.0
1.0 h
pSTAT5
STAT5
%
100
33
18
3.2
0.0
1.5 h
pSTAT5
STAT5
%
100
33
17
4.9
0.2
2.5 h
Average
(5 exps.)
%
100
42
20
4.0
0.0

Figure 7

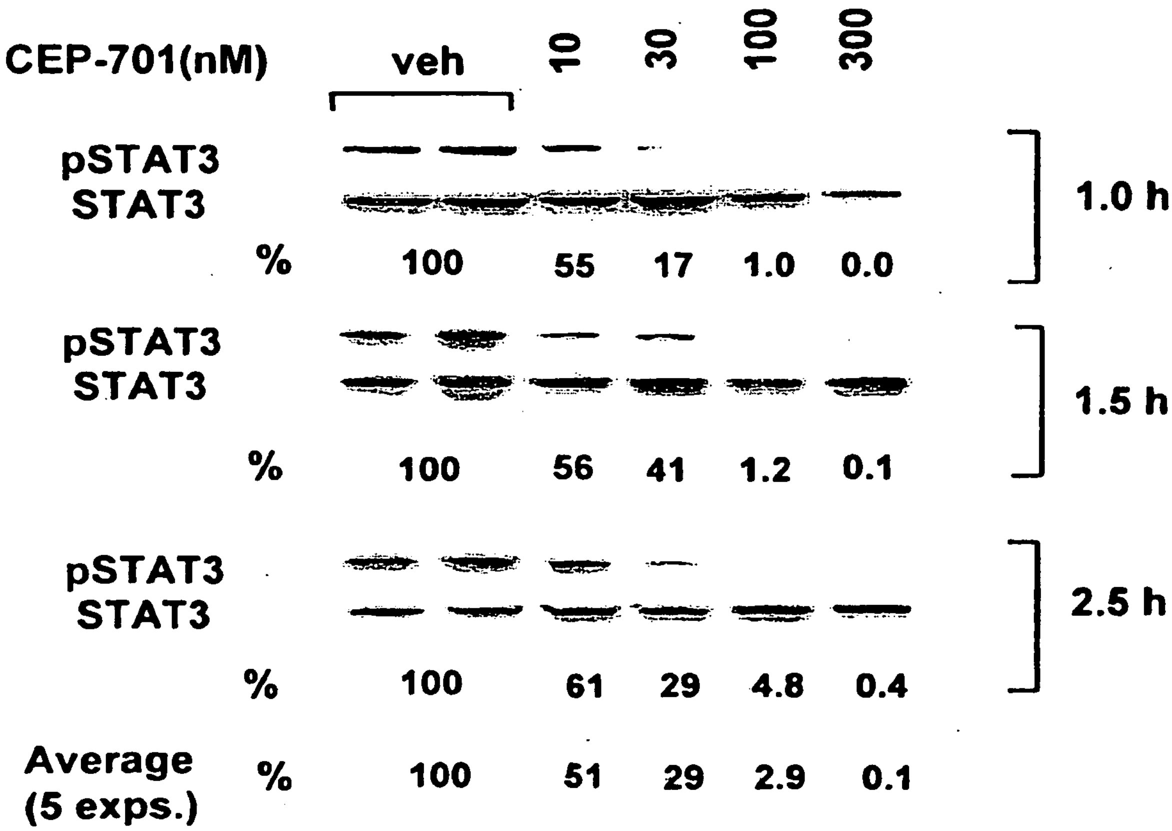
CEP-701(nM)
veh
10
30
100
300
pSTAT3
STAT3
1.0 h
%
100
55
17
1.0
0.0
pSTAT3
STAT3
1.5 h
%
100
56
41
1.2
0.1
pSTAT3
STAT3
2.5 h
%
100
61
29
4.8
0.4
Average
(5 exps.)
%
100
51
29
2.9
0.1

Figure 8

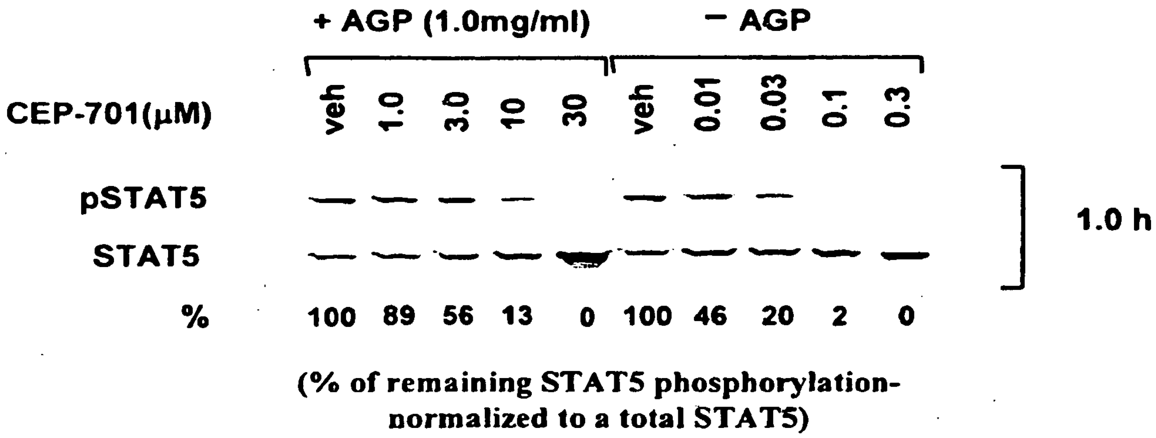
+ AGP (1.0mg/ml)
− AGP
CEP-701(μM)
veh
1.0
3.0
10
30
veh
0.01
0.03
0.1
0.3
pSTAT5
STAT5
1.0 h
%
100 89 56 13 0 100 46 20 2 0
(% of remaining STAT5 phosphorylation-normalized to a total STAT5)

Figure 9

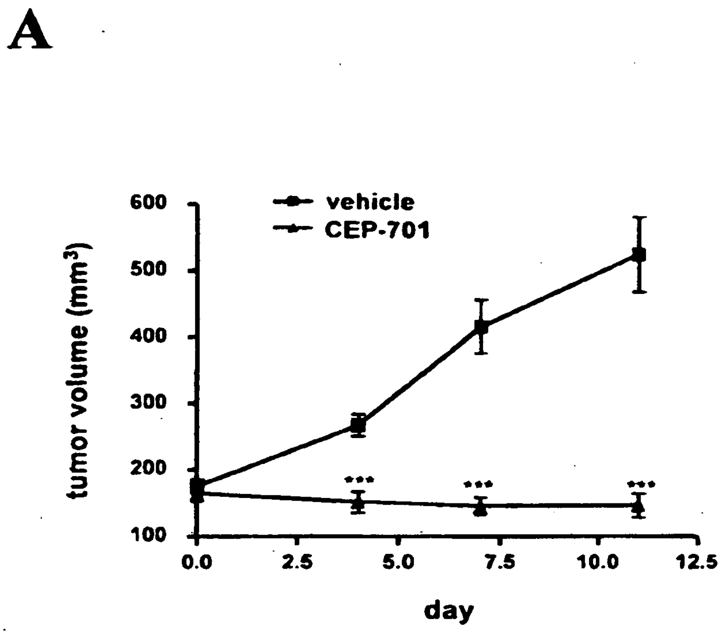
A
vehicle
CEP-701
tumor volume (mm3)
600
500
400
300
200
100
***
***
***
0.0
2.5
5.0
7.5
10.0
12.5
day

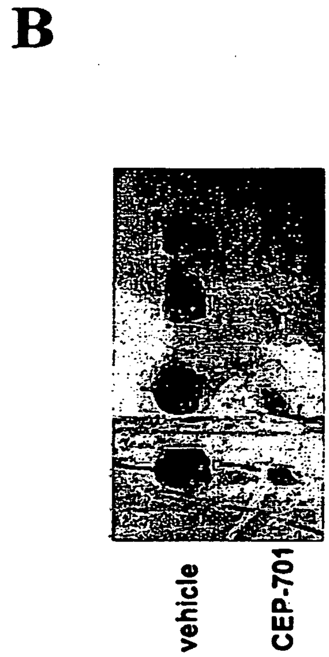
B
vehicle
CEP-701

Figure 10

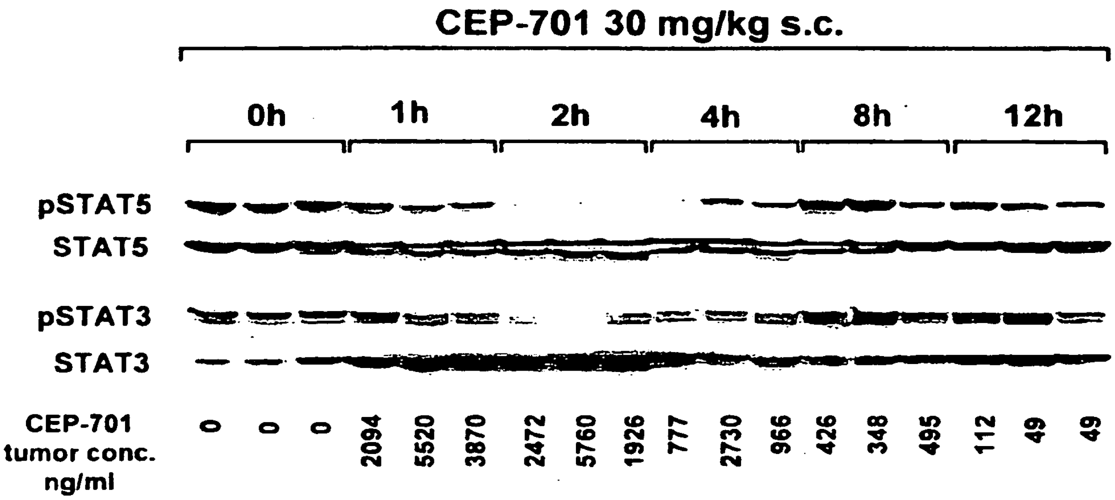
CEP-701 30 mg/kg s.c.
0h
1h
2h
4h
8h
12h
pSTAT5
STAT5
pSTAT3
STAT3
CEP-701 tumor conc. ng/ml
0 0 0 2094 5520 3870 2472 5760 1926 777 2730 966 426 348 495 112 49 49

Figure 11

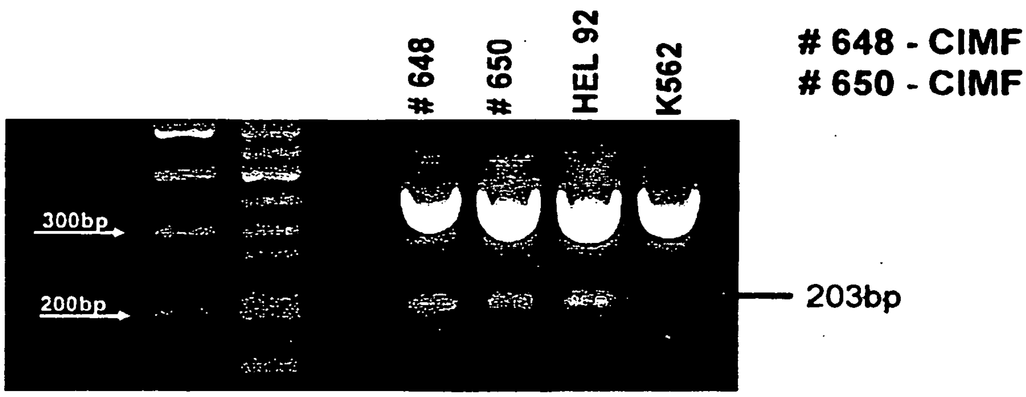


203bp = Mutant Allele
364bp = Mutant & Wild Type Allele (Internal Control)

**Figure 12**

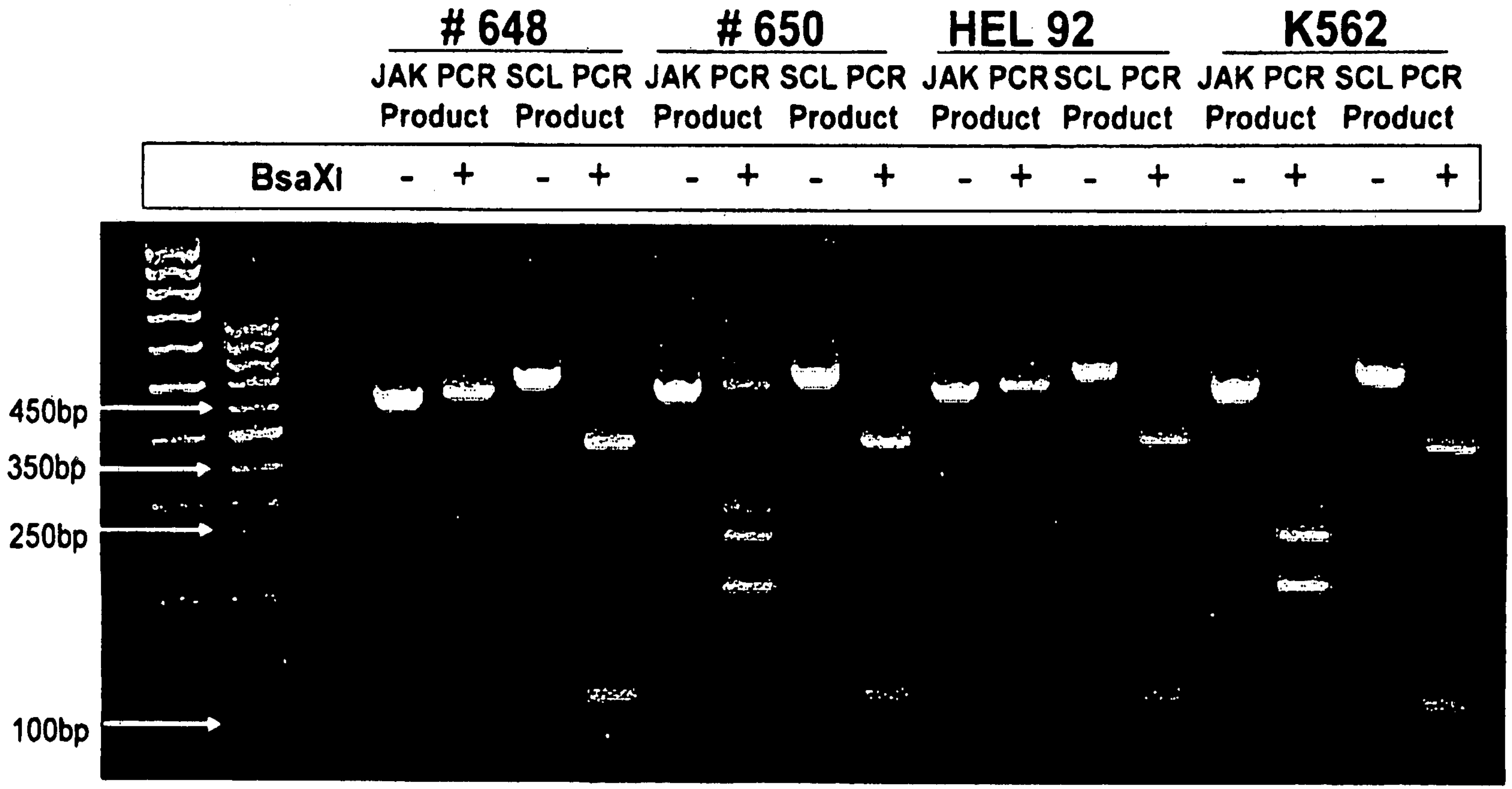


JAK2 PCR Product = 460bp

Mutant Allele = resistant to digestion with BsaXI

Wild Type Allele = 241bp, 189bp and 30bp restriction fragments

SCL PCR Product (control) = 496bp digested into 356, 110 and 30bp restriction fragments

**Figure 13**

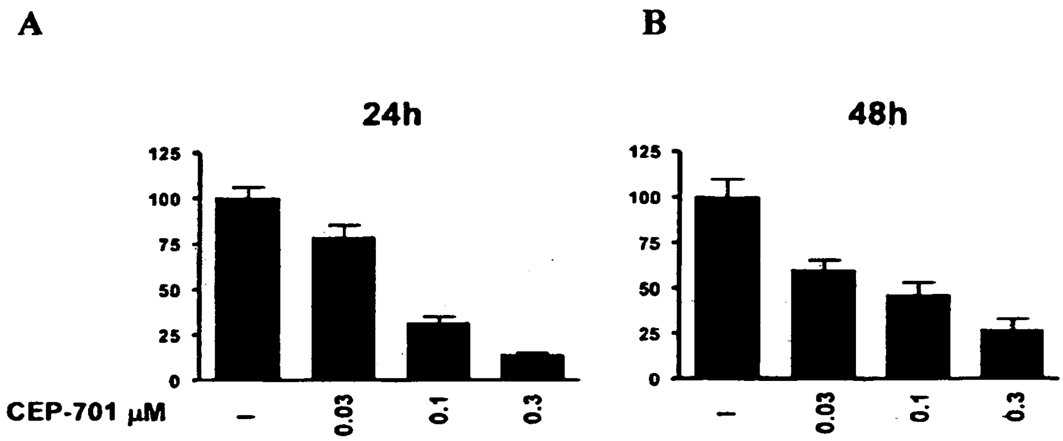


Figure 14

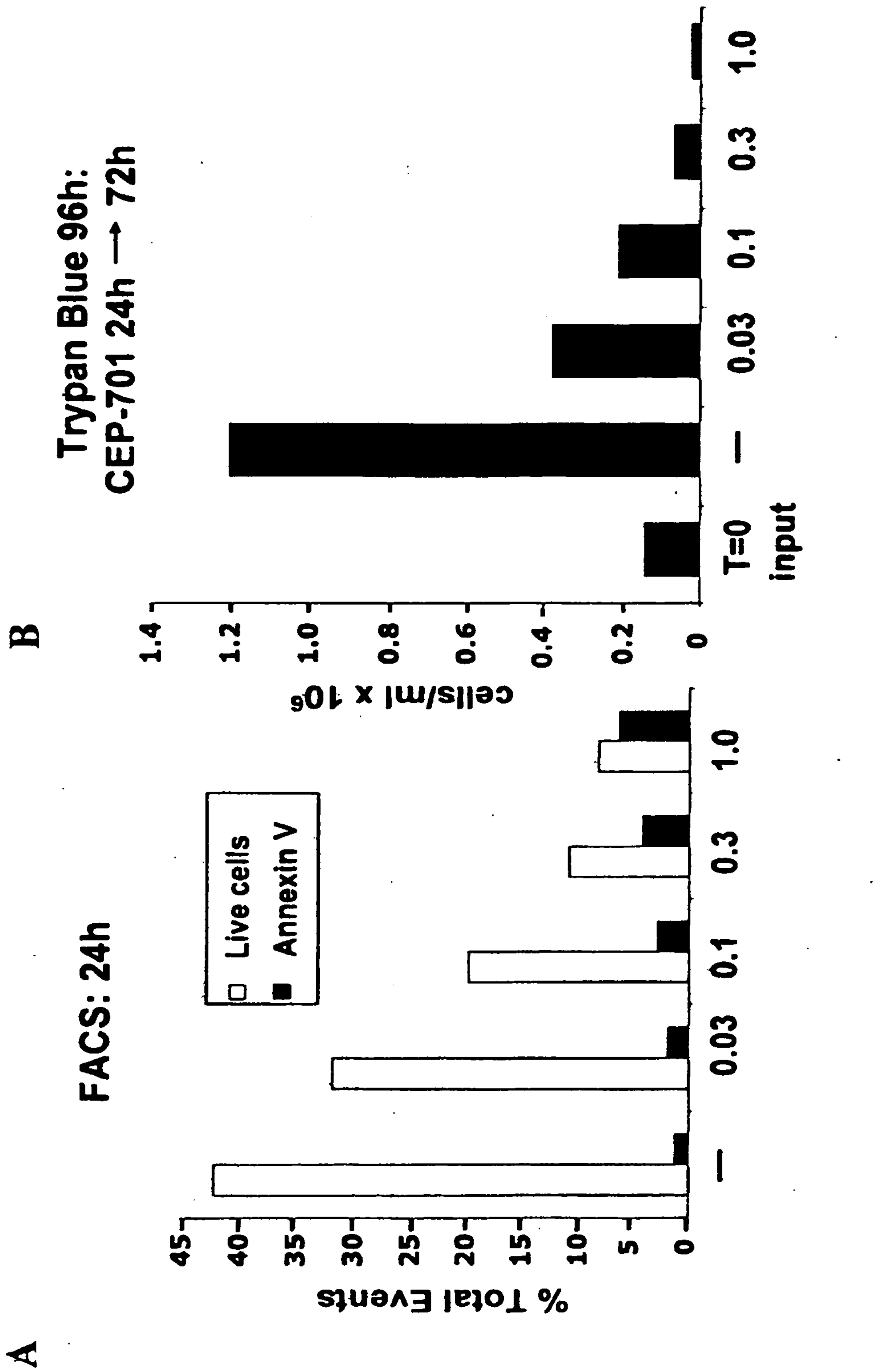
A
FACS: 24h
% Total Events
0
5
10
15
20
25
30
35
40
45
—
0.03
0.1
0.3
1.0
Live cells
Annexin V
B
Trypan Blue 96h:
CEP-701 24h → 72h
cells/ml x 10⁶
0
0.2
0.4
0.6
0.8
1.0
1.2
1.4
T=0
input
—
0.03
0.1
0.3
1.0

Figure 15

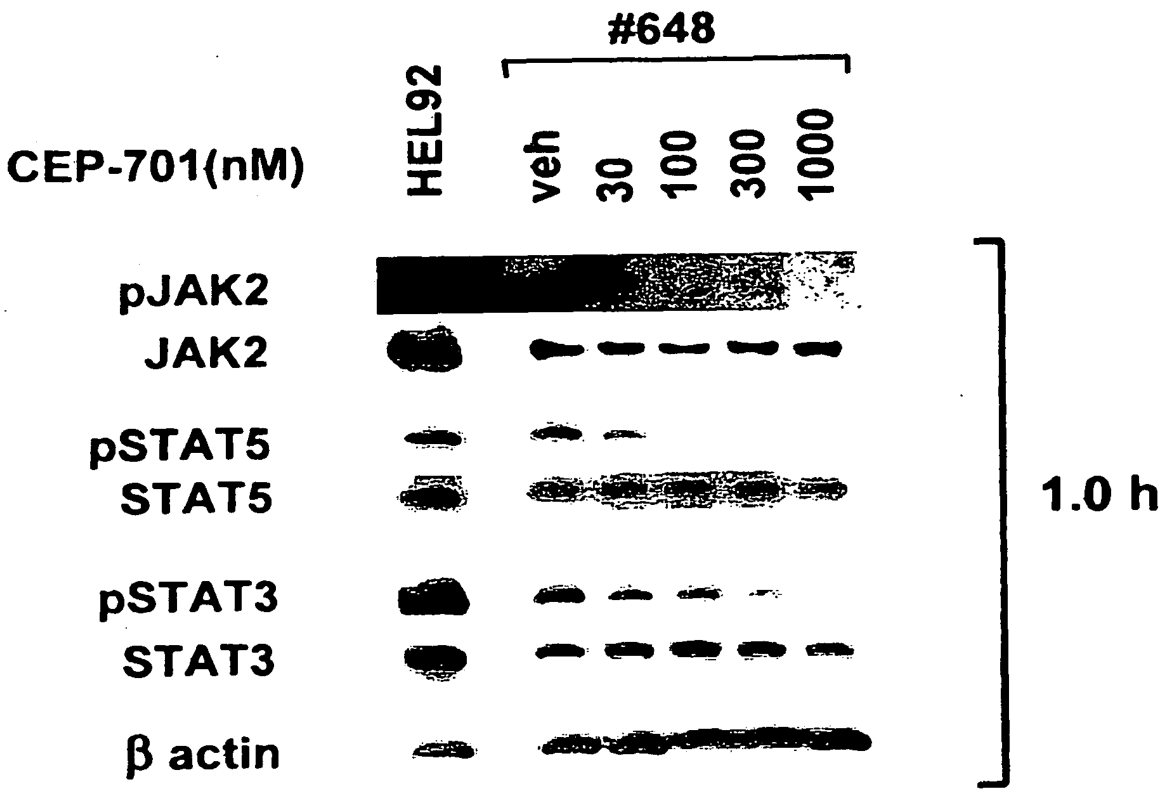
#648
CEP-701(nM)
HEL92
veh
30
100
300
1000
pJAK2
JAK2
pSTAT5
STAT5
pSTAT3
STAT3
β actin
1.0 h

Figure 16

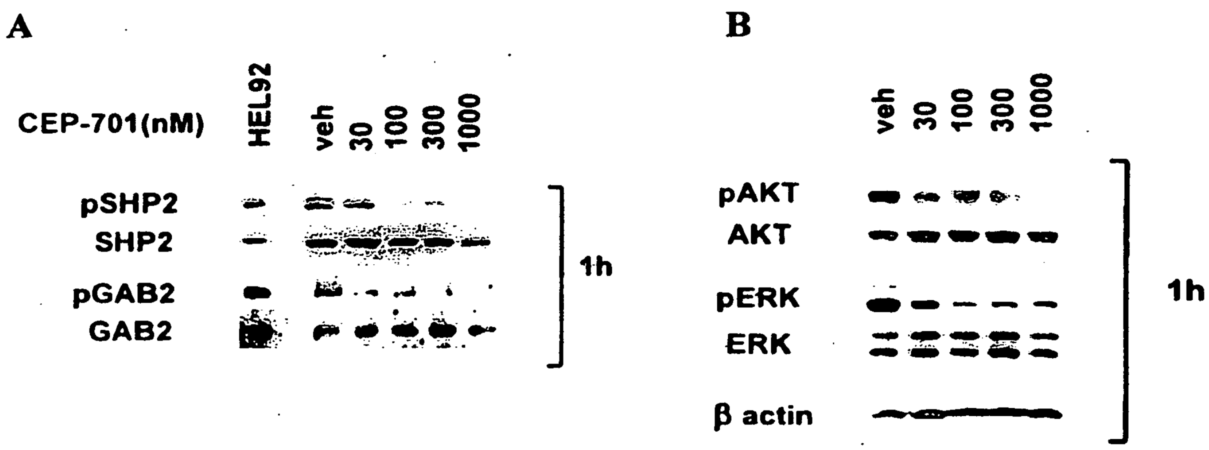
A
CEP-701(nM)
HEL92
veh
30
100
300
1000
pSHP2
SHP2
pGAB2
GAB2
1h
B
veh
30
100
300
1000
pAKT
AKT
pERK
ERK
β actin
1h

Figure 17

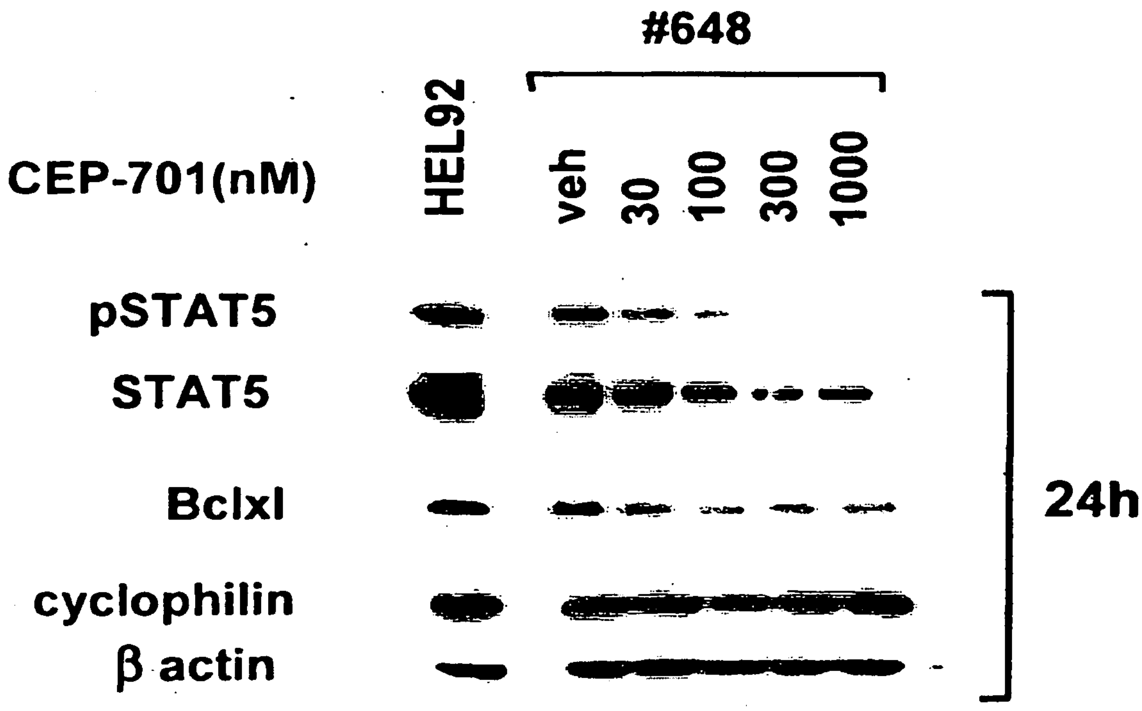
#648
CEP-701(nM)
HEL92
veh
30
100
300
1000
pSTAT5
STAT5
Bclxl
cyclophilin
β actin
24h

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4923986 A, Murakata
- US 5705511 A, Hudkins
- US 5808060 A, Hudkins
- US 6127401 A, Singh
- US 6841567 B
- US 6630500 B, Hudkins
- US 5756494 A, Lewis
- US 5468872 A, Glicksman
- US 5516771 A, Dionne
- US 6306849 B, Hudkins
- US 6093713 A, Hudkins


### Non-patent literature cited in the description


- **JAMES et al.** *Trends in Molecular Medicine,* 2005, vol. 11, 546-554
- **LEVINE.** *Cancer Cell,* 2005, vol. 7, 387-397
- **LEVINE et al.** *Cancer Cell,* 2005, vol. 7, 387-397
- **KRALOVICS et al.** *N. Engl. J. Med.,* 2005, vol. 352, 1779-1790
- **JAMES et al.** *Nature,* 2005, vol. 434, 1144-1148
- **WERNIG et al.** *Blood,* 14 February 2006
- **CAMPBELL et al.** *Blood,* 2006, 2098-2100
- **LEHNINGER.** Biochemistry. Worth Publishers, Inc, 1975, 73-75
- **BAXTER, E.J. et al.** Acquired mutation of the tyrosine kinase JAK2 in human myeloproliferative disorders. *Lancet,* 2005, vol. 365, 1054-1061